(19) Europäisches Patentamt
European Patent Office
Office européen des brevets

(11) EP 4 682 139 A1

(12) **EUROPEAN PATENT APPLICATION**
published in accordance with Art. 153(4) EPC

(43) Date of publication:
**21.01.2026 Bulletin 2026/04**

(21) Application number: **24770376.2**

(22) Date of filing: **16.02.2024**

(51) International Patent Classification (IPC):
***C07C 309/29*** (2006.01) ***C07C 303/42*** (2006.01)
***C07C 309/30*** (2006.01) ***C08F 12/30*** (2006.01)

(52) Cooperative Patent Classification (CPC):
**C07C 303/42; C07C 309/29; C07C 309/30; C08F 12/30**

(86) International application number:
**PCT/JP2024/005404**

(87) International publication number:
**WO 2024/190261 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.03.2023 JP 2023041575**
**06.11.2023 JP 2023189014**

(71) Applicants:
- **Tosoh Finechem Corporation**
  **Yamaguchi 746-0006 (JP)**
- **Sagami Chemical Research Institute**
  **Ayase-shi**
  **Kanagawa 252-1193 (JP)**

(72) Inventors:
- **OZOE, Shinji**
  **Shunan-shi, Yamaguchi 746-0006 (JP)**
- **SHIGETA, Yusuke**
  **Shunan-shi, Yamaguchi 746-0006 (JP)**
- **INOUE, Munenori**
  **Ayase-shi, Kanagawa 252-1193 (JP)**
- **YAMAZAKI, Manabu**
  **Ayase-shi, Kanagawa 252-1193 (JP)**

(74) Representative: **Michalski Hüttermann & Partner Patentanwälte mbB**
**Kaistraße 16A**
**40221 Düsseldorf (DE)**

# (54) AMMONIUM STYRENE SULFONATE COMPOSITION WITH EXCELLENT STORAGE STABILITY AND PRODUCTION METHOD THEREFOR

(57) To provide an ammonium styrenesulfonate composition excellent in long-term storage stability, and a simple and environmentally friendly method for producing the same.

An ammonium styrenesulfonate composition having characteristics (1) to (6) shown below:
(1) the ammonium styrenesulfonate composition has an ammonium styrenesulfonate content of 88.0% by weight or more;
(2) the ammonium styrenesulfonate composition has a water content of 10.00% by weight or less;
(3) the ammonium styrenesulfonate composition has an alkali metal content of 0.50% by weight or less;
(4) the ammonium styrenesulfonate composition has a halogen content of 1.00% by weight or less;
(5) the ammonium styrenesulfonate composition has a polymer content of 0.20% by weight or less; and
(6) the ammonium styrenesulfonate composition has a polymerization inhibitor content of 2000 ppm or less, and the method is used.

EP 4 682 139 A1

## Description

Technical Field

The present invention relates to an ammonium styrenesulfonate composition having excellent storage stability, in which reduction in purity and coloring due to spontaneous polymerization during long-term storage are suppressed, and a simple production method thereof.

Background Art

Sodium styrenesulfonate is a strong electrolyte-type water-soluble monomer having surface activity, and is used in a wide range of industrial fields because of its excellent heat resistance and radical polymerizability.

For example, sodium styrenesulfonate has long been used as a reactive emulsifier when producing an acrylic emulsion for an aqueous coating material or an aqueous adhesive. This is because when a radical polymerizable monomer such as an acrylic acid ester or a methacrylic acid ester is subjected to emulsion polymerization, the addition amount of a conventional emulsifier is reduced, and a small amount of sodium styrenesulfonate is added instead, and copolymerization is performed, thereby improving the colloid stability of the polymer emulsion and the water resistance and adhesion of the emulsion coating film.

However, when the added amount of sodium styrenesulfonate is increased in order to further enhance colloid stability, problems caused by sodium metal such as deterioration in water resistance of an emulsion coating film and corrosion of iron nails and the like used in wooden buildings may occur (for example, Non Patent Literature 1). Therefore, most of the anionic emulsifiers used in the production of the acrylic emulsion are nonmetallic ammonium salts (for example, Patent Literature 1 and Patent Literature 2).

Sodium polystyrenesulfonate, which is a polymer of sodium styrenesulfonate, is used in electronic material applications such as a carbon nanotube dispersant, a chemical mechanical polishing (CMP) slurry of a semiconductor substrate, and a cleaning agent after polishing (for example, Patent Literatures 3 to 5). However, in particular, in semiconductor applications, a metal component and a halogen component cause defects and corrosion of the substrate, and therefore it is required not to contain these components as much as possible (for example, Patent Literature 6).

Therefore, ammonium polystyrenesulfonate which does not contain a metal component or a halogen component as much as possible is more preferred.

From the above background, there is a strong market need for ammonium styrenesulfonate, and a production method thereof has been proposed in order to meet the market need (For example, Patent Literatures 7 and 8 and Non Patent Literature 2).

In Patent Literature 7, for example, sodium styrenesulfonate and ammonium sulfate are dissolved in methanol at 60°C and underwent a cation exchange reaction, and then the solution is cooled to 30°C to precipitate sodium sulfate generated by the cation exchange. Thereafter, the precipitated sodium sulfate is separated by filtration to recover a methanol solution of ammonium styrenesulfonate, and the methanol solution is further concentrated and dried to obtain an ammonium styrenesulfonate solid. It is described that this method utilizes a difference in solubility of ammonium styrenesulfonate and sodium sulfate generated by cation exchange in methanol.

However, since the total reactive substrate concentration is as low as about 9% by weight and it takes time to concentrate and dry, a polymer is easily generated during this time, and methanol used as a reaction solvent has toxicity and flammability, and therefore there is a problem in safety. Further, in this method, high purity ammonium styrenesulfonate was not necessarily obtained. This is because the lower the dielectric constant of the solvent, the lower the ion dissociation degree of the salt, and thus the cation exchange reaction is basically difficult to proceed in the organic solvent, and there is no sufficient solubility difference between sodium styrenesulfonate and ammonium styrenesulfonate with respect to the organic solvent, so that remaining sodium in ammonium styrenesulfonate cannot be avoided. In addition, industrially available sodium styrenesulfonate usually contains sodium bromide as an impurity in an amount of 2% by weight to 3% by weight. However, since sodium bromide and ammonium bromide generated by cation exchange between sodium bromide and ammonium sulfate are dissolved in methanol, remaining bromine in ammonium styrenesulfonate cannot be avoided.

In addition, ammonium styrenesulfonate obtained by the above method has a more serious problem in industrialization that storage stability is inferior to that of sodium styrenesulfonate. It was considered that an appropriate polymerization inhibitor was not added as a cause.

In Patent Literature 8, for example, paratoluidine hydrochloride is added to an aqueous solution of sodium styrenesulfonate, a styrenesulfonate toluidine salt precipitated by salt exchange is recovered, and then the toluidine salt is charged into an aqueous ammonia solution to be subjected to salt exchange again, thereby obtaining an aqueous solution of ammonium styrenesulfonate. However, mixing of toluidine into ammonium styrenesulfonate was inevitable because toluidine that is harmful and easily colored was used, and further, toluidine styrenesulfonate was solubilized in water by

ammonium styrenesulfonate. In addition, similarly to Patent Literature 7, when water is distilled off from an aqueous solution containing ammonium styrenesulfonate at a low concentration of about 10% by weight to precipitate ammonium styrenesulfonate crystals, a polymer is easily generated, and an appropriate polymerization inhibitor is not added, so that there is a large problem in long-term storage stability required for industrialization.

Non Patent Literature 2 describes that, for example, hydrogen chloride gas is blown into sodium styrenesulfonate dispersed in acetone to convert sodium styrenesulfonate into styrenesulfonic acid soluble in acetone and sodium chloride insoluble in acetone, and then sodium chloride is separated by filtration to recover an acetone solution of styrenesulfonic acid, and further neutralized with ammonia to obtain ammonium styrenesulfonate. However, there were problems: the use of highly flammable organic solvents and toxic hydrogen chloride gas and ammonia gas, and the unavoidable contamination of ammonium styrenesulfonate with ammonium chloride due to residual hydrogen chloride in the acetone solution of styrenesulfonic acid. As a more important problem, since styrenesulfonic acid as a reaction intermediate is very likely to undergo spontaneous polymerization (also referred to as self-initiated polymerization or non-catalytic polymerization), there is a problem that mixing of a polymer is inevitable (for example, Non Patent Literature 3).

From the above background, ammonium styrenesulfonate has not yet been industrialized, and there has been a strong demand for ammonium styrenesulfonate having both storage stability and high purity applicable to the above applications, and a simple and environmentally friendly production method thereof.

## Citation List

### Patent Literature

Patent Literature 1: JP 3585588 B2
Patent Literature 2: JP 3460246 B2
Patent Literature 3: JP 5482194 B2
Patent Literature 4: JP 6618355 B2
Patent Literature 5: JP 2021-44537 A
Patent Literature 6: WO 2020/184306 A
Patent Literature 7: JP 50-149642 A
Patent Literature 8: JP 51-26842 A

### Non Patent Literature

Non Patent Literature 1: Jose M.Asua; European Polymer Journal, Vol. 93, pp.480-494, 2017
Non Patent Literature 2: Toyo Soda Research Report, Vol. 24, No. 1, 1980, pp. 3-11
Non Patent Literature 3: J.C.Salamone; Polymer Letters Edition, Vol. 15, 1977, pp. 487-491

## Summary of Invention

### Technical Problem

The present invention has been made in view of the above background and problems, and an object of the present invention is to provide an ammonium styrenesulfonate composition having both long-term storage stability and high purity, and a simple and environmentally friendly production method for avoiding the use of harmful and dangerous organic solvents and gases.

### Solution to Problem

As a result of intensive studies by the present inventors, it has been found that the long-term storage stability as a conventional problem is significantly improved by controlling the moisture contained in the ammonium styrenesulfonate composition, the type and content of the polymerization inhibitor, and the specific metal component within specific ranges, and the ammonium styrenesulfonate composition having high purity and excellent long-term storage stability can be produced by performing reaction crystallization under specific conditions using an alkali metal styrenesulfonate, an inorganic ammonium salt, and a specific polymerization inhibitor and water as a solvent without using a harmful and dangerous organic solvent or gas and without undergoing strong acidic conditions under which a polymer is easily

generated, and the present invention has been completed. The styrenesulfonate referred to below is usually a para-form, but as is generally known, includes positional isomers such as a meta-form and an ortho-form.

That is, the present invention relates to the following.

[1] An ammonium styrenesulfonate composition having characteristics (1) to (6) shown below:

(1) the ammonium styrenesulfonate composition has an ammonium styrenesulfonate content of 88.0% by weight or more;
(2) the ammonium styrenesulfonate composition has a water content of 10.00% by weight or less;
(3) the ammonium styrenesulfonate composition has an alkali metal content of 0.50% by weight or less;
(4) the ammonium styrenesulfonate composition has a halogen content of 1.00% by weight or less;
(5) the ammonium styrenesulfonate composition has a polymer content of 0.20% by weight or less; and
(6) the ammonium styrenesulfonate composition has a polymerization inhibitor content of 2000 ppm or less.

[2] The ammonium styrenesulfonate composition according to item [1], having characteristics (1) to (6) shown below:

(1) the ammonium styrenesulfonate composition has an ammonium styrenesulfonate content of 88.0% by weight or more;
(2) the ammonium styrenesulfonate composition has a water content of 0.10% by weight to 10.00% by weight;
(3) the ammonium styrenesulfonate composition has an alkali metal content of 0.50% by weight or less;
(4) the ammonium styrenesulfonate composition has a halogen content of 1.00% by weight or less;
(5) the ammonium styrenesulfonate composition has a polymer content of 0.20% by weight or less; and
(6) the ammonium styrenesulfonate composition has a polymerization inhibitor content of 20 ppm to 2000 ppm.

[3] The ammonium styrenesulfonate composition according to item [1], having characteristics (1) to (6) shown below:

(1) the ammonium styrenesulfonate composition has an ammonium styrenesulfonate content of 94.00% by weight or more;
(2) the ammonium styrenesulfonate composition has a water content of 0.10% by weight to 6.00% by weight;
(3) the ammonium styrenesulfonate composition has an alkali metal content of 0.50% by weight or less;
(4) the ammonium styrenesulfonate composition has a halogen content of 0.10% by weight or less;
(5) the ammonium styrenesulfonate composition has a polymer content of 0.20% by weight or less; and
(6) the ammonium styrenesulfonate composition has a polymerization inhibitor content of 20 ppm to 1000 ppm.

[4] The ammonium styrenesulfonate composition according to any one of items [1] to [3], wherein the polymerization inhibitor is at least one selected from the group consisting of 2-methoxyphenol, 3-methoxyphenol, 4-methoxyphenol, 4-ethoxyphenol, 4-cyanophenol, 4-butoxyphenol, 3-ethoxyphenol, 2,5-dimethoxyphenol, 2,6-dimethoxyphenol, 2,6-di-tert-butylphenol, 2,4-di-tert-butylphenol, 2,6-di-tert-butyl-4-methylphenol, 4-tert-butylcatechol, hydroquinone, methylhydroquinone, 2-methoxyhydroquinone, tert-butylhydroquinone, ammonium N-nitrosophenylhydroxylamine, 4-hydroxy-2,2,6,6-tetramethylpiperidine 1-oxyl, 4-(2-hydroxypropoxy-3-(2-hydroxyethoxy))-2,2,6,6-tetramethylpiperidine-1-ol, 4-(3-hydroxypropoxy-2-(2-hydroxyethoxy))-2,2,6,6-tetramethylpiperidine-1-ol, and salicylic acid hydrazide.

[5] The ammonium styrenesulfonate composition according to any one of items [1] to [3], wherein the polymerization inhibitor is at least one phenolic compound selected from the group consisting of 2-methoxyphenol, 3-methoxyphenol, 4-methoxyphenol, 4-ethoxyphenol, 4-cyanophenol, 4-butoxyphenol, 3-ethoxyphenol, 2,5-dimethoxyphenol, and 2,6-dimethoxyphenol.

[6] The ammonium styrenesulfonate composition according to any one of items [1] to [3], wherein a charge amount per unit mass is 0.020 μC/g to 0.200 μC/g.

[7] The ammonium styrenesulfonate composition according to any one of items [1] to [3], wherein the median diameter of a crystal in the composition is from 30 μm to 700 μm.

[8] The ammonium styrenesulfonate composition according to any one of items [1] to [3], wherein the ammonium styrenesulfonate crystal has a median diameter of 30 μm to 500 μm.

[9] The ammonium styrenesulfonate composition according to any one of items [1] to [3], wherein the polymer content in the composition is 0.20% by weight or less when the composition is stored at 60°C for 60 days in a sealed state.

[10] The ammonium styrenesulfonate composition according to any one of items [1] to [3], wherein the composition has diffraction peaks at least at diffraction angles $2\theta = 8.1 \pm 0.2°$, $15.2 \pm 0.2°$, $18.4 \pm 0.2°$, $20.6 \pm 0.2°$, $24.2 \pm 0.2°$, $32.5 \pm 0.2°$, and $43.0 \pm 0.2°$ in a powder X-ray diffraction pattern measured by irradiation with copper Kα X-ray.

[11] The ammonium styrenesulfonate composition according to any one of items [1] to [3], wherein the composition

has diffraction peaks at least at diffraction angles $2\theta$ = 8.1 ± 0.2°, 15.2 ± 0.2°, 15.4 ± 0.2°, 18.4 ± 0.2°, 20.1 ± 0.2°, 20.6 ± 0.2°, 20.8 ± 0.2°, 24.2 ± 0.2°, 25.8 ± 0.2°, 27.5 ± 0.2°, 30.5 ± 0.2°, 32.5 ± 0.2°, 37.5 ± 0.2°, 43.0 ± 0.2° and 49.6 ± 0.2° in a powder X-ray diffraction pattern measured by irradiation with copper $K\alpha$ X-ray.

[12] The method for producing an ammonium styrenesulfonate composition according to any one of items [1] to [3], the method comprising bringing sodium or potassium styrenesulfonate into contact with an inorganic ammonium salt in water in the presence of a polymerization inhibitor in an amount of 7 mol% or less with respect to the amount of sodium or potassium styrenesulfonate to cause a cation exchange reaction, and then cooling to precipitate crystals of ammonium styrenesulfonate, and separating the crystals by filtration,
wherein

the charging ratio of ammonium cations to alkali metal styrenesulfonate is 1.50 equivalents to 3.00 equivalents, and
the total solids content in the reaction system is from 25.00% by weight to 50.00% by weight,
a temperature at which sodium or potassium styrenesulfonate is brought into contact with an inorganic ammonium salt is 30°C to 80°C, and
a temperature at which the crystals precipitated by cooling are separated by filtration is 5°C to 30°C.

[13] The method for producing the ammonium styrenesulfonate composition according to item [12],

the method comprising bringing sodium or potassium styrenesulfonate into contact with an inorganic ammonium salt in water in the presence of a polymerization inhibitor in an amount of 7 mol% or less with respect to the amount of sodium or potassium styrenesulfonate to cause a cation exchange reaction, and then cooling to precipitate crystals of ammonium styrenesulfonate, and separating the crystals by filtration,
wherein
the charging ratio of ammonium cations to alkali metal styrenesulfonate is 1.50 equivalents to 3.00 equivalents, and
the total solids content in the reaction system is from 25.00% by weight to 45.00% by weight,
a temperature at which sodium or potassium styrenesulfonate is brought into contact with an inorganic ammonium salt is 30°C to 80°C, and
a temperature at which the crystals precipitated by cooling are separated by filtration is 5°C to 30°C.

[14] The method for producing the ammonium styrenesulfonate composition according to item [12],

the method comprising bringing sodium or potassium styrenesulfonate into contact with an inorganic ammonium salt in water in the presence of a polymerization inhibitor in an amount of 5 mol% or less with respect to the amount of sodium or potassium styrenesulfonate to cause a cation exchange reaction, and then cooling to precipitate crystals of ammonium styrenesulfonate, and separating the crystals by filtration,
wherein
the charging ratio of ammonium cations to alkali metal styrenesulfonate is 2.00 equivalents to 2.50 equivalents, and
the total solids content in the reaction system is from 35.00% by weight to 45.00% by weight,
a temperature at which sodium or potassium styrenesulfonate is brought into contact with an inorganic ammonium salt is 40°C to 60°C, and
a temperature at which the crystals precipitated by cooling are separated by filtration is 5°C to 20°C.

[15] The method for producing an ammonium styrenesulfonate composition according to item [12], wherein the inorganic ammonium salt is at least one compound selected from the group consisting of ammonium chloride, ammonium sulfate, ammonium nitrate, and ammonium acetate.

## Advantageous Effects of Invention

The ammonium styrenesulfonate composition of the present invention can be produced without using dangerous and harmful raw materials and an unstable intermediate, and the decrease in purity and coloring during long-term storage, which is the obstacle to industrialization, are remarkably reduced by optimizing the composition, and thus, is extremely useful for the production of an acrylic emulsion and the production of an ammonium styrenesulfonate polymer for electronic materials. In addition, since the ammonium styrenesulfonate composition of the present invention is dissolved in a polar organic solvent, it is extremely useful for production of an electrolyte membrane and modification of a polymer substrate by graft polymerization.

Brief Description of Drawings

Fig. 1 is a schematic diagram of a wet crystal of AmSS (composition).

Fig. 2 is a proton nuclear magnetic resonance spectrum chart of an AmSS composition obtained in Example 1. The horizontal axis represents a chemical shift (ppm), and the four decimal places numerical values at the bottom of the chart indicate the integral ratio of protons bonded to each carbon atom. Ha to He in the structural formula correspond to Ha to He in the vicinity of each peak, and a peak around 3 ppm corresponds to a methyl proton of dimethyl sulfone added as an internal standard.

Fig. 3 is an optical microscope photograph of an AmSS composition obtained in Example 1 (photographing magnification: 100x). In the photograph, the scale indicated by the blue line represents 250 μm.

Fig. 4 is an optical microscope photograph of an AmSS composition before drying obtained in Example 5 (photographing magnification: 100x). In the photograph, the scale indicated by the blue line represents 250 μm.

Fig. 5 is a proton nuclear magnetic resonance spectrum chart of AmSS obtained in Comparative Example 11, and the numerical values in the figure are the same as those in Fig. 2.

Fig. 6 is an optical microscope photograph of AmSS obtained in Comparative Example 11 (photographing magnification: 200x). In the photograph, the scale indicated by a white line represents 50 μm.

Fig. 7 is a view showing a powder X-ray diffraction pattern of an AmSS wet crystals obtained in Example 5. The vertical axis represents the diffraction intensity (unit: counts), the horizontal axis represents the diffraction angle 2θ (unit: degree), and the numerical value at the top of each peak in the drawing represents the detection angle of the peak top.

Fig. 8 is an enlarged view of Fig. 7 (enlarged range on the horizontal axis: $10° \leq 2\theta \leq 30°$). The numerical values in the drawing are the same as those in Fig. 7.

Fig. 9 is a view showing a powder X-ray diffraction pattern of AmSS dried crystals (wet crystals dried by a rotary evaporator) obtained in Example 5. The numerical values in the drawing are the same as those in Fig. 7.

Fig. 10 is an enlarged view of Fig. 9 (enlarged range on the horizontal axis: $10° \leq 2\theta \leq 30°$). The numerical values in the drawing are the same as those in Fig. 7.

Fig. 11 is a view showing a powder X-ray diffraction pattern of NaSS as a raw material used in Example 5. The numerical values in the drawing are the same as those in Fig. 7.

Fig. 12 is an enlarged view of Fig. 11 (enlarged range on the horizontal axis: $10° \leq 2\theta \leq 30°$). The numerical values in the drawing are the same as those in Fig. 7.

Fig. 13 is a view showing a powder X-ray diffraction pattern of AmSS dried crystals (wet crystals dried by a rotary evaporator) obtained in Example 6. The numerical values in the drawing are the same as those in Fig. 7.

Fig. 14 is an enlarged view of Fig. 13 (enlarged range on the horizontal axis: $10° \leq 2\theta \leq 30°$). The numerical values in the drawing are the same as those in Fig. 7.

Description of Embodiments

Hereinafter, modes for carrying out the present invention (Hereinafter, it is referred to as "the present embodiment".) will be described in detail. Note that the present invention is not limited to the following embodiment. The present invention can be appropriately modified and implemented within the scope of the gist thereof.

First, characteristics of the ammonium styrenesulfonate (Hereinafter, it is abbreviated as AmSS.) composition of the present invention will be described in detail.

Sodium styrenesulfonate (Hereinafter, it is abbreviated as NaSS.), which is a raw material of the AmSS composition of the present invention, is a powdered vinyl monomer having extremely excellent storage stability, and

no decrease in purity or coloration due to spontaneous polymerization is observed at least for 3 to 4 years after production at normal temperature storage. NaSS is produced by reacting 4-(2-bromoethyl) benzenesulfonic acid with sodium hydroxide in water as shown in the following flow, but the anhydrous salt (anhydrous crystal) has a problem that the crystal powder is solidified in about half a year to one year at normal temperature after production, or the purity is lowered by spontaneous polymerization.

Subsequently, it was found that the long-term storage stability was dramatically improved by inducing a crystal transition of the anhydrous salt of NaSS to convert it into the hemihydrate salt form (hemihydrate crystal) (see, for example, JP 10-152465 A). The NaSS currently in circulation is a hemihydrate salt with improved storage stability, and forms stable crystals with two molecules of NaSS and one molecule of water, but actually, since the attached water (2% by weight to 3% by weight) derived from the slurry filtrate (mother liquor) is included in addition to the crystal water (theoretical value: 4.4% by weight), the total moisture in the product NaSS is usually 6% by weight to 8% by weight. During the above reaction, sodium nitrite or the like is added as a polymerization inhibitor, and a nitrite component of about 20 ppm to 100 ppm remains in the NaSS product.

[Chemical formula 1]

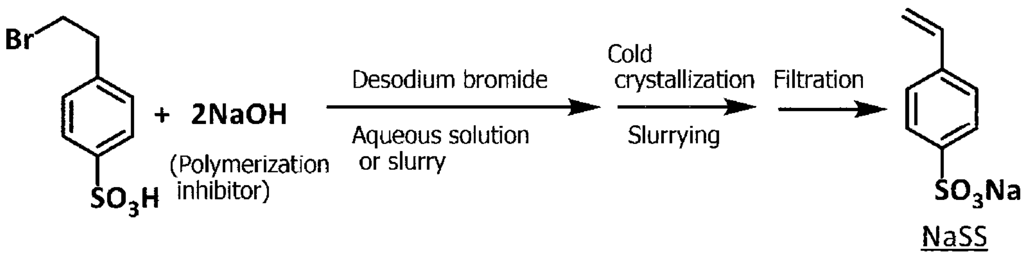


On the other hand, since ammonia is weak in basicity and AmSS cannot be produced directly from 4-(2-bromoethyl) benzenesulfonic acid and ammonia, attempts have been made to produce AmSS from commercially available NaSS as described above. However, as described above, there are large problems in the production process such as the use of dangerous and harmful raw materials, via an unstable intermediate, a large number of steps, and a reaction at a low substrate concentration, and industrialization has not been achieved.

Therefore, the present inventors have studied a simple and highly productive method for producing AmSS using only water as a reaction solvent. As a result, the present inventors have found that when industrialized lithium styrenesulfonate (Hereinafter, it is abbreviated as LiSS.), an inorganic ammonium salt, and a small amount of lithium nitrite and sodium nitrite are dissolved in water with a specific composition and then cooled, AmSS is surprisingly preferentially crystallized and slurried, and AmSS can be extremely easily acquired by filtering the slurry.

Since the solubility of AmSS in water is only about half of the solubility of LiSS (see homepage of Tosoh Finechem Corporation (https://www.tosoh-finechem.co.jp)), it is considered that AmSS is preferentially crystallized, and unreacted LiSS and other inorganic salts having high solubility remain in the mother liquor. However, the AmSS has poor storage stability, and there is a problem that the AmSS is lowered in purity or colored due to spontaneous polymerization 1 to 2 months after preparation even when stored at a low temperature. As a cause, it was suggested that nitrite, which acted as a stabilizer for NaSS and LiSS, might not act on AmSS.

Therefore, the present inventors examined in detail the factors affecting the storage stability of the AmSS. As a result, it has been found that the following components (1) to (3) and the amounts thereof are important, and by controlling these components within a specific range, it is possible to significantly suppress a decrease in purity and coloring due to polymerization during long-term storage.

(1) The content of water in the AmSS composition,
(2) the type and content of polymerization inhibitors in the AmSS composition, and
(3) remaining metal species and content in AmSS composition

Hereinafter, the characteristics of the AmSS composition of the present invention will be described in more detail.

That is, (1) the content of water in the AmSS composition is 10.00% by weight or less, and the lower the content, the more preferable it is from the viewpoint of storage stability and fluidity, more preferably 6.00% by weight or less, and particularly preferably 5.00% by weight or less. On the other hand, as the content of water is lower, the composition is more likely to be charged, the risk of scattering and dust explosion during handling work is increased, and a long-time drying step

is required in order to reduce the moisture as much as possible, so that the content of water in the composition is practically 0.10% by weight or more.

(2) The content of the polymerization inhibitor contained in the AmSS composition depends on the type of the polymerization inhibitor, but is usually 20 ppm or more, and more preferably 100 ppm or more. When the content of the polymerization inhibitor is less than 20 ppm, sufficient stability may not be obtained. On the other hand, when the content is too large, the polymerization rate, polymerization degree, and hue of AmSS when the AmSS composition is used may be adversely affected, and therefore the content is 2000 ppm or less, more preferably 1000 ppm or less, still more preferably 500 ppm or less.

(3) The coloring mechanism of the AmSS composition is not necessarily clear, but it is presumed that interaction of an alkali metal such as lithium metal, nitrite, a phenolic polymerization inhibitor, and the like is involved. At least the contents of lithium and nitrite are preferably as low as possible, and the content of lithium is preferably 20 ppm or less, usually 1 ppm or less, and the nitrite component is preferably 20 ppm or less. The nitrite component referred to herein is basically derived from sodium nitrite or lithium nitrite contained in NaSS and LiSS as a raw material, and is a nitrite anion that can be quantified by ion chromatography or the like.

In the present invention, the purity of the AmSS composition is preferably 88.00% by weight or more, further 94.00% by weight or more, and particularly 95.00% by weight or more. However, since water is a main impurity, if the AmSS composition is forcibly dried or washed with a water-soluble organic solvent such as alcohol or acetone to reduce moisture, the purity is inevitably improved.

The polymerization inhibitor is not particularly limited as long as it is soluble in water or a reaction solution and suppresses spontaneous polymerization of AmSS, and examples of the polymerization inhibitor include phenol-based polymerization inhibitors such as 2-methoxyphenol, 3-methoxyphenol, 4-methoxyphenol, 4-ethoxyphenol, 4-cyanophenol, 4-butoxyphenol, 3-ethoxyphenol, 2,6-dimethoxyphenol, 2,5-dimethoxyphenol, 4-isopropoxyphenol, 1,4-dihydroxy-2-methoxybenzene, hydroquinone, methyl hydroquinone, 2-methoxyhydroquinone, and 2,4-dinitrophenol; semi-hindered phenol-based polymerization inhibitors such as 4-tert-butylcatechol, butylhydroxyanisole, and tert-butylhydroquinone; hindered phenol polymerization inhibitors such as 2,6-di-tert-butylphenol, 2,4-di-tert-butylphenol, 2,6-di-tert-butyl-4-methylphenol, and 2,5-di-tert-butylhydroquinone; stable nitroxyl radical polymerization inhibitors such as 4-hydroxy-2,2,6,6-tetramethylpiperidine 1-oxyl, 4-(2-hydroxypropoxy-3-(2-hydroxyethoxy))-2,2,6,6-tetramethylpiperidin-1-ol, and 4-(3-hydroxypropoxy-2-(2-hydroxyethoxy))-2,2,6,6-tetramethylpiperidin-1-ol; and polymerization inhibitors such as ammonium N-nitroso-N-phenylhydroxylamine, L-ascorbic acid, erythorbic acid, catechin, tocopherol and urea. Among them, 2-methoxyphenol, 3-methoxyphenol, 4-methoxyphenol, 4-ethoxyphenol, 4-cyanophenol, 4-butoxyphenol, 3-ethoxyphenol, 2,5-dimethoxyphenol, 2,6-dimethoxyphenol, which are the same aromatic compounds as those of AmSS, and 4-hydroxy-2,2,6,6-tetramethylpiperidine 1-oxyl, 4-(2-hydroxypropoxy-3-(2-hydroxyethoxy))-2,2,6,6-tetramethylpiperidine-1-ol, and 4-(3-hydroxypropoxy-2-(2-hydroxyethoxy))-2,2,6,6-tetramethylpiperidine-1-ol, which have a high polymerization inhibiting ability, which are more preferable from the viewpoint of compatibility with AmSS, solubility during the reaction, and coloring resistance. Nitrite-based polymerization inhibitors such as sodium nitrite and lithium nitrite used in producing NaSS and LiSS may adversely affect the coloring resistance of AmSS, and thus it is preferable to avoid them.

In addition, the charged charge amount per unit mass of the AmSS composition of the present invention is 0.200 μC/g or less. As the charged charge amount per unit mass is larger than 0.200 μC/g, the AmSS composition is more likely to be charged, and the scattering properties and the dust explosibility at the time of handling work are enhanced. It is most important to secure storage stability, but by controlling the moisture of the AmSS composition within the above range, both low chargeability and high storage stability can be achieved.

In addition, the median diameter of the AmSS crystal in the AmSS composition is preferably 30 μm to 700 μm from the viewpoint of securing hygroscopicity resistance, high fluidity, low dust generation properties, and dust explosibility resistance although it does not directly affect the spontaneous polymerizability and coloring properties of the AmSS. As will be described later, it is preferable that the crystal size of AmSS is large and the thickness is large because the deliquoring properties are good and the purity of AmSS is improved. However, when the crystal size is excessively large, the deliquoring properties may be rather reduced, and therefore the crystal size is more preferably 500 μm or less. On the other hand, when the median diameter is less than 30 μm, the deliquoring properties are significantly deteriorated. In addition, even if the crystal size of AmSS after filtration or drying is large, when the AmSS is stirred and mixed or loosened using a crusher or the like, crystals or aggregates of crystals are broken and reduced, but from the viewpoint of suppressing hygroscopicity, dust generation properties, and dust explosibility, the median diameter is preferably maintained at 30 μm or more.

When the AmSS composition of the present invention is stored in a sealed state at least at 60°C for 60 days, the polymer content in the composition is 0.20% by weight or less, and the storage stability is extremely excellent due to the control of moisture and the presence of an appropriate polymerization inhibitor. In addition, when the AmSS composition is

colored by oxidation of AmSS or a polymerization inhibitor or the like, it is not preferable in applications where hue is important, such as paints and adhesives. For example, the AmSS composition of the present invention stored in a sealed state at 60°C for 60 days preferably has an APHA value of 100 or less as a 10% by weight aqueous solution.

The content of water in the AmSS composition can be quantified by a $^1$H-NMR method, a Karl Fischer moisture meter, a dry weight method using a constant temperature dryer, an infrared moisture meter, or the like. Among them, the infrared moisture meter is the most convenient and has good reproducibility.

The ammonium styrenesulfonate content contained in the AmSS composition, that is, the purity can be quantified by quantitative analysis of active vinyl groups by redox titration (see, for example, JP 2014-80505 A and paragraph 0055), proton nuclear magnetic resonance spectroscopy ($^1$H-NMR method), high performance liquid chromatography (HPLC), or the like.

In the case of quantitative determination by $^1$H-NMR, for example, the AmSS composition and a compound such as dimethylsulfone as an internal standard are precisely weighed, dissolved in a deuterated solvent such as deuterated dimethyl sulfoxide, and $^1$H-NMR is measured. Then, the ammonium styrenesulfonate component in the AmSS composition can be quantified from the ratio of the integral value of methyl protons of dimethylsulfone to the integral value of protons derived from the styrenesulfonic acid skeleton, for example, protons of vinyl groups. However, since the alkali metal styrenesulfonate and the ammonium styrenesulfonate cannot be distinguished from each other by any of the methods described above, it is necessary to confirm the molar ratio of the ammonium cation to the styrenesulfonic acid unit and the metal component by elemental analysis or $^1$H-NMR.

In the present invention, the crystal shape of the resulting AmSS composition is usually a substantially circular or square plate shape, and the shape and size thereof can be measured with an optical microscope or an electron microscope. However, in the present invention, a median diameter that can be easily and reproducibly measured with a laser diffraction/scattering type particle size distribution meter is used. The diffraction/scattering type particle size distribution meter calculates a particle size by regarding a specimen as spherical particles, and the median diameter is a diameter in which when the specimens are divided into two from a certain particle size, the larger side and the smaller side are equal in amount.

In addition, the content of the polymerization inhibitor of the AmSS composition can be quantified by gas chromatography (GC), high performance liquid chromatography (HPLC), ion chromatography (IC), or the like depending on the type of the polymerization inhibitor.

The alkali metal and halogen which may be contained in the AmSS composition of the present invention are impurities derived from raw materials, and it is preferable that the alkali metal and halogen are as low as possible in consideration of use in an aqueous coating material. Usually, the alkali metal content in the composition is preferably 0.50% by weight or less, and the halogen content is preferably 1.00% by weight or less. In consideration of use in an electronic material, the alkali metal content in the composition is more preferably 0.50% by weight or less, and the halogen content is more preferably 0.10% by weight or less. In addition, when the AmSS composition is dissolved in a polar organic solvent to produce a polymer, the larger the amount of alkali metal such as sodium, the more likely the polymer is to be precipitated during polymerization, and therefore the lower the amount is, the better. These metal components can be quantified using high-frequency inductively coupled plasma analysis (ICP-AES) or the like. Impurities such as halogen can be quantified using ion chromatography (IC), a Volhard method, or the like.

A polymer that may be contained in the AmSS composition of the present invention is an impurity derived from raw materials or generated during production or storage. When AmSS is used for production of an acrylic emulsion, colloid stability and physical properties of an emulsion coating film are deteriorated as the polymer content in the AmSS composition increases, and therefore the content is preferably as low as possible, preferably 0.20% by weight or less, and more preferably 0.10% by weight or less.

In addition, when a solid compound such as AmSS is actually used on an industrial scale, AmSS is often dissolved in water or an organic solvent and filtered, but filterability is dramatically deteriorated due to the presence of a polymer component, and thus it is preferable that the polymer component is as low as possible. In particular, when AmSS is dissolved in an organic solvent and filtered, not only a polymer component but also inorganic impurities such as a sodium component and an inorganic ammonium salt deteriorate filterability, and therefore it is preferable that the inorganic impurities are as small as possible.

In addition, the charge amount per unit mass of the AmSS composition can be measured by a small charging evaluation device of an air transport system (Teruo Suzuki; Proceedings of the Institute of Electrostatics Japan, No. 25, vol. 1, pp. 37-44, 2001), an electrolytic flying type charge amount measuring device (DIT Inc.), an E-SPART analyzer (Keisuke Tsuji; Pulverization, No. 5, pp. 84-88, 2014), or the like.

Hereinafter, a method for producing the AmSS composition of the present invention will be described.

As described above, the present inventors have succeeded in finding a composition for retaining long-term storage stability without impairing the polymerizability when using the AmSS composition, and therefore, next, conducted intensive studies on a simple method for producing the AmSS composition. The targeted process is simply represented in the following flow. That is, the point based on the cation exchange reaction between the alkali metal styrenesulfonate and

the inorganic ammonium salt is the same as that in the related art.

[Chemical formula 2]

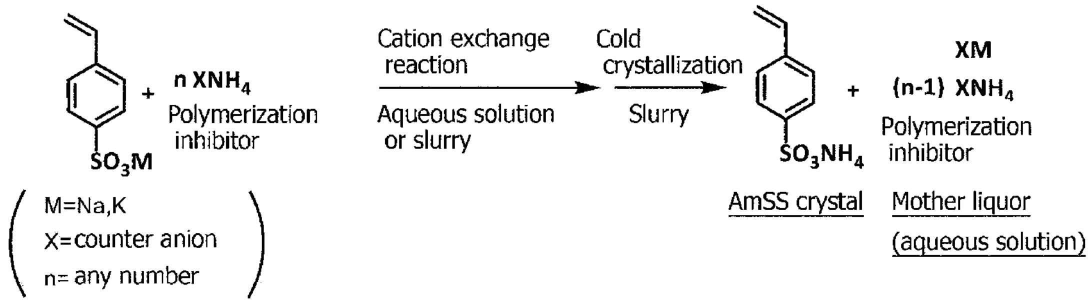


As described above, although AmSS can be easily obtained by a cation exchange reaction between LiSS and an inorganic ammonium salt, AmSS is preferentially crystallized because the solubility of LiSS in water is about twice higher than that of AmSS. On the other hand, in the production method of the present invention, NaSS and potassium styrenesulfonate (hereinafter, it is abbreviated as KSS) having lower solubility in water than AmSS are used, and it is usually unlikely that high-purity AmSS can be obtained under such conditions. However, as a result of examination by the present inventors, it has been found that AmSS is preferentially crystallized under surprisingly high substrate concentration conditions. That is, it was found that crystals of AmSS can be obtained only by filtering the aqueous slurry liquid shown in the above flow.

By the way, since AmSS is a strong electrolyte-type hydrophilic compound, attachment of the mother liquor (filtrate) cannot be avoided, and the AmSS accurately becomes a wet crystal (composition) as shown in the schematic diagram of Fig. 1. Then, the amount of impurities in the AmSS composition is determined by the amount of the mother liquor containing a high-concentration inorganic salt attached. It would be preferable to be able to grow an AmSS crystal having a low amount of the mother liquor attached, that is, having excellent deliquoring properties in the reaction system, but it has been found that a high total substrate concentration to some extent is required in order to obtain a high-quality AmSS crystal. That is, since a high substrate concentration is synonymous with a high salt concentration in the mother liquor, the impurity concentration in the AmSS composition does not decrease even if the deliquoring properties are improved. The present inventors have found a unique production condition capable of achieving both storage stability and high purity of AmSS.

The reason why AmSS is preferentially crystallized under the production conditions of the present invention is not necessarily clear, but it is estimated as follows.

First, water having a high dielectric constant is used as a reaction solvent instead of an organic solvent, and 1.50 equivalents or more of ammonium cations are added to NaSS (sodium styrenesulfonate) and KSS (potassium styrenesulfonate). Since the ion dissociation degree of both is high, it can be said that at least the cation exchange reaction is likely to proceed.

Secondly, a difference in cation size is considered. That is, in general, the size of the lithium cation is smaller than that of the sodium cation, and the size of the sodium cation is smaller than that of the ammonium cation, but it is known that the smaller the cation size is, the more easily hydration occurs. For example, when LiSS, NaSS, and AmSS are actually crystallized from an aqueous solution, LiSS easily forms extremely fine needle-like crystals, and thus the deliquoring properties are poor, and the amount of attached water exceeds well 20% by weight. On the other hand, NaSS forms large circular plate-shaped crystals, and AmSS easily forms large quadrangular plate-shaped crystals. Therefore, the deliquoring properties are better than that of LiSS, and the amount of attached water is at least less than 20% by weight, and depending on crystallization conditions, the amount of attached water may be reduced to several % by weight. In particular, it has been found that AmSS easily forms a large and thick crystal. The above facts are believed to be related to the reason why high purity AmSS compositions can be obtained under the conditions of the present invention.

Hereinafter, the manufacturing method will be described more specifically. First, water, an alkali metal styrenesulfonate, an inorganic ammonium salt, and a polymerization inhibitor are charged into a reactor at a specific composition, and the raw materials are dissolved or partially dissolved while being stirred at a specific temperature for a specific time. Thereafter, crystals of AmSS are precipitated and grown while being cooled to a predetermined temperature at a specific speed. Subsequently, the precipitated AmSS crystals are separated by filtration to obtain the AmSS composition of the present invention.

As the alkali metal styrenesulfonate to be used, a sodium salt and a potassium salt can be used. In addition, an alkaline earth metal styrenesulfonate such as calcium styrenesulfonate can also be used, but a mass-produced sodium

salt is more preferable.

Examples of the inorganic ammonium salt include ammonium chloride, ammonium bromide, ammonium sulfate, ammonium nitrate, ammonium phosphate, ammonium dihydrogen phosphate, diammonium hydrogen phosphate, and ammonium acetate, and in consideration of economic efficiency such as price, ammonium chloride, ammonium sulfate, ammonium nitrate, ammonium phosphate, ammonium dihydrogen phosphate, and diammonium hydrogen phosphate are more preferable. Furthermore, ammonium chloride, ammonium sulfate, and ammonium nitrate are more preferable in consideration of solubility of an alkali metal salt produced as by-products in the cation exchange. Furthermore, ammonium sulfate and ammonium nitrate are more preferable in consideration of residual halogen. However, even when AmSS is produced using ammonium chloride, a high-purity AmSS composition containing less impurities such as halogen can be produced by purification by the method described later.

The polymerization inhibitor is as described above.

First, the composition to be charged in the reaction will be described.

The charging ratio of the ammonium cation to the alkali metal styrenesulfonate is preferably 1.50 equivalents to 3.00 equivalents. When the equivalent ratio is less than 1.50 equivalents, the cation exchange rate is low, and the salt concentration in the system decreases, so that it may be difficult to generate high-quality AmSS crystals having a good deliquoring properties. On the other hand, even when the equivalent ratio exceeds 3.00 equivalents, the deliquoring properties are not improved, and instead, the inorganic salt concentration in the attached mother liquor increases, so that the purity of the AmSS composition may decrease. From the viewpoint of the balance between the concentration of the inorganic salt in the mother liquor and the deliquoring properties, 2.00 equivalents to 2.50 equivalents is more preferable.

The total solids content in the reaction system is preferably 25.00% by weight to 50.00% by weight. When the total solids content is less than 25.00% by weight, not only the yield decreases, but also the deliquoring properties decrease. On the other hand, when the total solids content is more than 50.00% by weight, the inorganic salt concentration in the mother liquor increases, and the purity of the AmSS composition may decrease. The total solids content is preferably 25.00% by weight to 45.00% by weight, more preferably 30.00% by weight to 45.00% by weight, even more preferably 35.00% by weight to 45.00% by weight from the viewpoint of the balance between the concentration of the inorganic salt in the mother liquor and the deliquoring properties.

Here, the total solids content means the sum of raw materials that are present in the reaction system and are solid at room temperature, impurities that are contained in the raw materials and are solid at room temperature, and by-products that are solid at room temperature.

The reaction temperature is preferably 30°C to 80°C. In the composition to be charged, when the reaction temperature is lower than 30°C, the cation exchange rate may be insufficient, and when the reaction temperature is higher than 80°C, the cation exchange rate is improved, but a polymer may be easily produced, and therefore the temperature is more preferably 30°C to 60°C, still more preferably 40°C to 60°C.

The reaction time is 10 minutes to 20 hours, and can be adjusted according to the substrate concentration and the reaction temperature, but is preferably 30 minutes to 20 hours, further 10 minutes to 10 hours, particularly 30 minutes to 10 hours in order to prevent spontaneous polymerization during the reaction.

The cooling and filtration temperatures are 5°C to 30°C, and after reaching a predetermined cooling temperature, aging is further performed for 0.5 hours to 5 hours. When the cooling temperature is lower than 5°C, impurities such as the inorganic salt and moisture of the AmSS composition may increase, and when the cooling temperature is higher than 30°C, the yield may significantly decrease. Therefore, the cooling temperature is more preferably 5°C to 25°C, and still more preferably 5°C to 20°C.

The cooling rate can be adjusted according to the heating temperature and the total solids content, but is preferably 3°C per hour to 40°C per hour. When the cooling rate is too fast, the crystal size decreases, and the deliquoring properties may deteriorate. As a result, moisture and the inorganic salt of the AmSS composition may increase. On the other hand, when the cooling rate is extremely slow, the crystal may collapse and the deliquoring properties may deteriorate, and therefore the cooling rate is more preferably 5°C per hour to 30°C per hour.

In order to increase the crystal size of AmSS and improve the deliquoring properties, so-called temperature swing crystallization is more preferable, in which after the crystal is precipitated by cooling to a predetermined temperature, the crystal is reheated to a temperature at which all the crystals are not dissolved, and cooled again. When each raw material is charged into the reactor, the raw material may be charged as a powder, or may be charged as a saturated aqueous solution of each raw material.

In addition, the method of charging may be either batch charging or sequential charging. When an aqueous solution or a saturated aqueous solution of an inorganic ammonium salt is added dropwise to an aqueous solution or a slurry liquid of alkali metal styrenesulfonate, the entrapment (inclusion) of the inorganic salt into precipitated AmSS crystals is reduced, so that a higher-quality AmSS composition can be obtained. In addition, a powder or a slurry liquid of alkali metal styrenesulfonate may be added to the aqueous solution of the inorganic ammonium salt, but it is disadvantageous in terms of scattering of the powder and sedimentation properties of the slurry liquid. The inside of the reaction system may be either an inert atmosphere or an air atmosphere, but an air atmosphere is more preferable from the

viewpoint of suppressing spontaneous polymerization during the reaction.

In addition, in order to increase the crystal size of AmSS to enhance the deliquoring properties, AmSS can be added as a seed crystal from the start of the reaction during cooling. The addition amount of the seed crystal is preferably 1.0 mol% to 20.0 mol% with respect to the styrenesulfonic acid unit in the system, and more preferably 1.0 mol% to 10.0 mol% in consideration of productivity and thermal history.

The molar ratio of the ammonium cation charged to the alkali metal styrenesulfonate defined by the production method of the present invention varies depending on the addition of the AmSS seed crystal, but the ammonium cation mentioned herein is limited to those derived from the inorganic ammonium salt charged as a raw material, and does not include the ammonium cation derived from the seed crystal.

In order to increase the yield of the resulting AmSS, a water-soluble organic solvent that is a poor solvent may be added to the reaction system. Examples of the solvent include alcohols such as methanol, ethanol, and 2-propanol, ketones such as acetone, nitriles such as acetonitrile, and ethers such as tetrahydrofuran. From the viewpoint of an increase in inorganic impurities in the AmSS composition, environmental load, and explosion-proof measures, a solvent of water alone is preferable as an industrial method.

For filtration of the slurry, methods such as centrifugal filtration, pressure filtration, reduced pressure filtration, and filter press can be applied, but centrifugal filtration that has a large treatment capacity and can treat in a relatively short time is more preferable. Further, impurities can be further reduced by recrystallizing and purifying the obtained AmSS composition using water or a mixed solvent of water and the above-described water-soluble organic solvent. At this time, from the viewpoint of productivity, the total solids content in the system is preferably 50.00% by weight to 60.00% by weight, the heating temperature is preferably 40°C to 60°C, and the cooling and filtration temperature is preferably 10°C to 25°C. When recrystallization and purification are performed, it is preferable to add 7 mol% or less of the above-mentioned polymerization inhibitor based on AmSS.

In addition, since the storage stability of the AmSS composition is better as moisture is lower, the water content of the AmSS composition can be reduced by washing the AmSS composition with the above-described water-soluble organic solvent. In addition, the AmSS composition may be dried using a shelf tier vacuum dryer, a conical stirring dryer (Nauta mixer), a vacuum rotary dryer (conical dryer), a rotary kiln dryer, a spray dryer, a pleat dryer, a double cylindrical dry filter, a vacuum vibration dryer, or the like. However, depending on drying conditions such as a heating temperature, ammonia volatilizes and the degree of ammonia neutralization of styrenesulfonic acid decreases, and the storage stability of the AmSS composition may rather decrease. Therefore, the degree of ammonia neutralization may be maintained at 100% as much as possible.

As a result of close examination by the present inventors, it has been found that commercial NaSS is stabilized by a hemihydrate salt crystal as described above, but the AmSS composition of the present invention is stabilized by an anhydrous salt crystal. That is, commercially available moisture of NaSS is composed of crystal water and attached water, and in order to remove these, a high temperature and a degree of pressure reduction to some extent are required, but since moisture of the AmSS composition is attached water, these can be removed even at a lower temperature and degree of pressure reduction. For example, it can be dried with dry air at room temperature or an inert gas stream. The drying temperature is preferably 20°C to 70°C, and more preferably 20°C to 40°C in consideration of volatilization of the polymerization inhibitor and polymerization of AmSS.

Since the AmSS composition of the present invention is soluble in water, it can be used for production of an aqueous polymer emulsion, hollow polymer particles, an aqueous solution of ammonium polystyrenesulfonate, and the like, and since it is also soluble in a polar organic solvent, it is extremely useful for production of a polymer electrolyte membrane (For example, US 6221248) and surface modification of an organic material using graft polymerization (for example, Kyoichi Saito, Polymer Adsorbent by Graft Polymerization, pp. 8-10, published by Maruzen Publishing Co., Ltd., 2014; NHV Corporation Homepage https://www.nhv.jp/blog/post723/).

X-ray powder diffraction (PXRD) patterns can be measured by common protocols. As described above, it is presumed that the AmSS composition of the present invention is stabilized by anhydrous salt crystals, and the PXRD pattern is also attributed to this.

The AmSS composition of the present invention has diffraction peaks at least at a diffraction angle $2\theta$ of 8.1 $\pm$ 0.2°, 15.2 $\pm$ 0.2°, 15.4 $\pm$ 0.2°, 18.4 $\pm$ 0.2°, 20.1 $\pm$ 0.2°, 20.6 $\pm$ 0.2°, 20.8 $\pm$ 0.2°, 24.2 $\pm$ 0.2°, 25.8 $\pm$ 0.2°, 27.5 $\pm$ 0.2°, 30.5 $\pm$ 0.2°, 32.5 $\pm$ 0.2°, 37.5 $\pm$ 0.2°, 43.0 $\pm$ 0.2° and 49.6° $\pm$ 0.2, and in particular, has strong diffraction peaks at a diffraction angle $2\theta$ of 8.1 $\pm$ 0.2°, 15.2 $\pm$ 0.2°, 18.4 $\pm$ 0.2°, 20.6 $\pm$ 0.2°, 24.2 $\pm$ 0.2°, 32.5 $\pm$ 0.2° and 43.0 $\pm$ 0.2°. This is a PXRD pattern that is clearly distinct from sodium styrenesulfonate, which is stabilized by hemihydrate salt crystals. Typical errors in peak position are $\pm$0.2°, and these minor errors can occur due to sample preparation, measuring instruments, measurer handling, etc. As for the value of the diffraction peak, for example, "8.2 $\pm$ 0.2°" means that the diffraction peak is present in any of the range of 8.0° to 8.4°(that is, 8.0° $\leq 2\theta \leq$ 8.4°).

Examples

The present invention will be described more specifically with reference to the following Examples, but the present invention is not limited by these Examples at all.

<Drug to be used>

Sodium styrenesulfonate (NaSS): manufactured by Tosoh Finechem Corporation, purity 88.2%, sodium bromide 2.2% by weight, sodium hydroxide 0.40% by weight, sodium sulfate 0.50% by weight, moisture 7.2% by weight, polymer component 0.01% by weight, and nitrite component 70 ppm

Lithium styrenesulfonate (LiSS): manufactured by Tosoh Finechem Corporation, purity 85.3%, lithium bromide 2.5% by weight, lithium hydroxide 0.45% by weight, lithium sulfate 0.50% by weight, moisture 7.1% by weight, polymer component 0.04% by weight, and nitrite component 60 ppm
Ammonium chloride: manufactured by FUJIFILM Wako Pure Chemical Corporation, special grade reagent
Ammonium sulfate: manufactured by FUJIFILM Wako Pure Chemical Corporation, special grade reagent
Ammonium nitrate: manufactured by FUJIFILM Wako Pure Chemical Corporation, special grade reagent
Diammonium hydrogen phosphate: manufactured by FUJIFILM Wako Pure Chemical Corporation, special grade reagent
4-Methoxyphenol: manufactured by FUJIFILM Wako Pure Chemical Corporation, special grade reagent
2-Methoxyphenol: manufactured by FUJIFILM Wako Pure Chemical Corporation, special grade reagent
4-Ethoxyphenol: manufactured by Tokyo Chemical Industry Co., Ltd., purity >98%
2,6-Dimethoxyphenol: manufactured by Tokyo Chemical Industry Co., Ltd., purity >98%
Methylhydroquinone: manufactured by Tokyo Chemical Industry Co., Ltd., purity >98%
4-t-Butylcatechol: manufactured by FUJIFILM Wako Pure Chemical Corporation, special grade reagent
4-hydroxy-2,2,6,6-tetramethylpiperidine 1-oxyl: manufactured by Tokyo Chemical Industry Co., Ltd., purity >98%
Lithium nitrite: 40% by weight aqueous solution, manufactured by Honjo Chemical Corporation
Phenolic antioxidant emulsion (Antage3LX): Kawaguchi Chemical Industry Co., Ltd.
2,2'-Azobis(2-methylpropionamidine)dihydrochloride (V-50): manufactured by FUJIFILM Wako Pure Chemical Corporation, first-grade reagent)
Dimethylsulfone: manufactured by FUJIFILM Wako Pure Chemical Corporation, special grade reagent
N-Methylpyrrolidone: manufactured by FUJIFILM Wako Pure Chemical Corporation, special grade reagent

<Quantification of AmSS moisture>

Measurement was performed under the following conditions using an infrared moisture meter.
Apparatus: FD-720 manufactured by Kett Electric Laboratory Co. Ltd.
Condition: About 5 g of a sample was collected and heated at 120°C for 20 minutes.

<Purity Analysis of AmSS by redox titration>

The active double bond was quantified by the following redox titration method to obtain AmSS purity (that is, in addition to the para-form, an ortho-form and a meta-form are also included).

(1)Device and apparatus

1) Weighing bottle: diameter 50 mm, depth 70 mm
2) 500 ml, 1000 ml Volumetric flask
3) 500 ml Erlenmeyer flask with stopper
4) Electrochemical balance

(2) Reagents

1) Bromine liquid: 22.00 g of potassium bromide (KBr) and 3.00 g of potassium bromate ($KBrO_3$) were dissolved in pure water to make the total 1000 ml.
2) Aqueous sulfuric acid solution (concentrated sulfuric acid/pure water volume ratio = 1/1)
3) Aqueous potassium iodide solution (200 g/L)
4) 0.1 mol/L aqueous sodium thiosulfate solution

5) Aqueous starch solution: 6.00 g of starch was dissolved in pure water to make the total 1000 ml.

(3) Operation

1) Into a weighing bottle, 20 g of a sample is weighed to the order of 0.1 mg.
2) The sample is washed and transferred to a 500 ml volumetric flask with pure water to make the liquid volume approximately 400 ml.
3) A magnetic rotor is placed and stirred to dissolve the sample.
4) The rotor is taken out, and fill to the mark with purified water, shake well to mix, and use the solution as the test solution.
5) To a 500 ml-stoppered Erlenmeyer flask containing 200 ml of pure water, 25 ml of a bromine liquid is added.
6) After 5 ml of the test solution is added, 10 ml of an aqueous sulfuric acid solution is added, and the resulting mixture is hermetically sealed and allowed to stand for 20 minutes.
7) Aqueous potassium iodide solution 10 ml is quickly added and allowed to stand for 10 minutes.
8) Titration is performed with an aqueous sodium thiosulfate solution. After the yellow color of the solution becomes light, 1 ml of a starch solution is added as an indicator, and the titration is performed until the blue color of the resulting iodine starch disappears.
9) Separately, as a blank test, 200 ml of pure water is added, 25 ml of a bromine liquid is added to a stoppered Erlenmeyer flask, 10 ml of an aqueous potassium iodide solution and 10 ml of an aqueous sulfuric acid solution are quickly added, and the operation of 8) is performed.

(4) Calculation

The AmSS content is calculated by the following equation.

$$A = 100 \times [0.01006 \times (a - b) \times f]/(S \times 5/500)$$

A: AmSS content (%)
a: aqueous sodium thiosulfate solution required for blank test (ml)
b: aqueous sodium thiosulfate solution required in the present test (ml)
f: titer of aqueous sodium thiosulfate solution
S: sample amount (g)

<Elemental analysis of AmSS>

Carbon, hydrogen and nitrogen components were quantified using an element analyzer
Apparatus: 2400II manufactured by PerkinElmer Co., Ltd.

<Quantification of sulfur component of AmSS>

The combustion gas of the AmSS composition burned by the oxygen flask combustion method was absorbed into the hydrogen peroxide absorbent to prepare an absorbent. Next, the sulfate ion concentration in the absorption was quantified by ion chromatography (see the next item for measurement conditions) and converted into a sulfur component.

<Quantification of bromine, chlorine, sulfuric acid, nitric acid and nitrite anions in AmSS>

Quantification was performed under the following conditions using ion chromatography.

Apparatus: IC-2010 manufactured by Tosoh Corporation
Column: TSKgel (registered trademark) guard column Super IC-AHS(4.6 mmI.D. ×1 cm) +TSKgel (registered trademark) SuperIC-Anion HS(4.6 mmI.D. ×10 cm)
Column temperature: 40°C, injection volume: 30 μl, flow rate: 1.5 ml/min
Eluent: carbonate buffer (7.5 mM-$NaHCO_3$ + 0.8 mM-$Na_2CO_3$)
Preparation of sample solution: The AmSS composition was dissolved in ultrapure water and diluted 10 times, and passed through a pretreatment cartridge (TOYOPAK (registered trademark) ODSM) to obtain a measurement sample.

Calibration curve: absolute calibration curve method using standard solution

<Analysis of AmSS by proton nuclear magnetic resonance ($^1$H-NMR)>

The molar ratio of AmSS component, moisture, and ammonium cation to styrenesulfonic acid unit was analyzed under the following conditions.

(1) Sample preparation

The sample was dissolved in about 0.7 mL of dimethylsulfoxide-d6 (99.5% by weight) containing about 0.05% by weight of tetramethylsilane as an internal standard substance to prepare a measurement sample.

(2) Measuring instruments and conditions

Model = AV-400M manufactured by Bruker
Number of integrations = 16
Nuclide =1H

(3) Calculation of molar ratio of ammonium cation ($NH_4$)/styrenesulfonic acid unit (SS)

Calculation was performed by the following equation.

$$NH_4/SS = [n/(nH)]/[s/(sH)]$$

s: integral value of peak derived from vinyl group of SS (peak position: 55.30 ppm, d)
n: integral value of peak derived from NH4 (peak position: δ7.22 ppm, s, provided that it may be divided into 3 depending on moisture)
sH: number of hydrogens in peak of s (= 1)
nH: number of hydrogens in peak of n (= 4)

Incidentally, the peak position is when dimethylsulfoxide-d6 is used as a heavy solvent, and the chemical shift is slightly changed depending on the amount of impurities.

(4) Calculation of molar ratio of moisture ($H_2O$)/styrenesulfonic acid unit (SS)

Calculation was performed by the following equation.

$$H_2O/SS = [m/(mH)]/[s/(sH)]$$

s: integral value of peak derived from vinyl group of SS (peak position: 55.30 ppm, d)
m: integral value of peak derived from H2O (peak position: δ3.40 ppm, s)
sH: number of hydrogens in peak of s (= 1)
mH: number of hydrogens in the peak of m (= 2)

Since dimethyl sulfoxide-d6 may contain moisture, moisture in dimethyl sulfoxide-d6 was quantified in advance, and the integral value was corrected.

(5) Calculation of AmSS component

The AmSS component in the AmSS composition was calculated by the following equation.

AmSS component (wt%) = (B/Mb) × (a/aH)/(b/bH) × Ma/S × 100

a: integral value of peak derived from vinyl group of AmSS (peak position: δ5.30 ppm, d)
b: integral value of peak derived from internal standard substance dimethyl sulfone (peak position: δ3.00 ppm, s)
aH: number of hydrogens in the peak of a (= 1)
bH: number of hydrogens in the peak of b (= 6)
Ma: molecular weight of AmSS
Mb: molecular weight of internal standard substance
B: collection amount of internal standard (g)
S: collection amount of sample (g)

<Analysis of alkali metal component of AmSS>

Sodium and lithium components were quantified using a frequency inductively coupled plasma emission spectrometer OPTIMA 8300 (manufactured by PerkinElmer, Inc.) (Hereinafter, it is abbreviated as ICP-AES.). The target sample was wet-decomposed with sulfuric acid and nitric acid, and the decomposed sample was heated to dryness. Nitric acid was added to the dried sample, and the dried sample was heated and dissolved, the dried sample was made up to the specified volume, and then scandium was added in an amount of 1 ppm as an internal standard for quantification.

<Analysis of polymer component of AmSS and polymerization inhibitor>

Analysis was performed under the following conditions using gel permeation chromatography (GPC).

Apparatus: HLC-8320 manufactured by Tosoh Corporation
Column: TSKgel (registered trademark) guard column AW-H/TSKgel (registered trademark) AW-6000/TSKgel (registered trademark) AW-3000/TSKgel (registered trademark) AW-2500
Eluent: 0.05M aqueous sodium sulfate solution/acetonitrile = 65/35 (volume ratio) solution
Sample: A 1% by weight solution of the AmSS composition (neat form) was prepared using the eluent.
Flow rate/injection amount/column temperature: 0.6 ml/min, injection amount: 10 μl, column temperature: 40°C
Detector: UV detector (wavelength: 230 nm)
Calibration curve: standard solutions having different sodium polystyrenesulfonate concentrations were prepared using sodium polystyrenesulfonate (manufactured by Sowa Kagaku Co., Ltd., 3K) and the eluent, and a calibration curve was prepared. All absorption peaks with an elution time of less than 13.00 minutes were regarded as polymer components.

For the polymerization inhibitor, a calibration curve was prepared in the same manner.

<Measurement of polymerization conversion rate and molecular weight during AmSS polymerization>

Measurement was performed by GPC under the same conditions as above (however, the sample concentration is 0.1% by weight). A calibration curve was prepared from the peak top molecular weight of standard sodium polystyrenesulfonate (manufactured by Sowa Kagaku Co., Ltd., 3 K, 15 K, 41 K, 300 K, 1000 K, 2350 K, and 5000 K) and the elution time, and automatically calculated with internal software, and the polymerization conversion rate was calculated from the following formula from the peak area (a) of the monomer and the peak area (b) of the polymer.

$$\text{Conversion (area\%)} = 100 \times [1 - \{a/(a + b)\}]$$

<Confirmation of long-term storage stability of AmSS>

The AmSS was sealed in a glass sample bottle and aged in an oven at 60°C for a predetermined time, and then the sample was returned to normal temperature, and the APHA value and the polymer component were measured. The APHA value was under the same conditions as described below, and the polymer component was measured by the same GPC as described above (sample concentration: 1% by weight).

<Analysis of hue of AmSS>

APHA value: The chrominance meter (ZE-6000 manufactured by Nippon Denshoku Industries Co., Ltd.) was powered on and stabilized for 30 minutes, and then the measurement method was set to transmission and the light source/field was set to C/2. Water was placed in a square cell (cell length: 36 mm), standard calibration was performed,

then the AmSS aqueous solution prepared to have a concentration of 10% by weight (based on neat form) was transferred to the square cell and set, and the APHA value was measured.

<Measurement of median diameter of AmSS>

Measurement was performed under the following conditions using a Microtrac particle size analyzer (MT-3300EXII manufactured by Nikkiso Co., Ltd.). About 0.5 g of the AmSS composition was put into 200 ml of hexane to obtain a sample liquid, the sample liquid was irradiated with a laser beam, and diffraction (scattering) thereof was measured to determine the particle size. In the preparation of the sample liquid, a treatment such as an ultrasonic wave was not performed, and measurement was performed under the setting conditions of particle permeability = transmission, particle shape = non-spherical, particle refractive index = 1.81, and solvent refractive index = 1.38. When the moisture of the AmSS composition was more than 10% by weight, dispersibility in hexane was deteriorated. Therefore, the moisture of the AmSS composition was previously dried to 10% by weight or less using a rotary evaporator and used for sample preparation.

<Measurement of charged charge amount of AmSS (empty transmission method)>

The sample was charged into the powder supply part of the charging tube, allowed to stand for 10 minutes, and then the sample was transported with air at a flow rate of 130 L/min, and the charged charge amount on the charging tube side was calculated from the voltage value obtained from the Faraday gauge and the current value obtained from the charging tube.

Electrometer: Model6514 (manufactured by KEITHLEY)
Charging tube: 24.6φ × 500 mm made of SUS
Measurement temperature: 25 to 26°C (humidity 20 to 30%)
Sample amount: about 0.50 g

<X-ray diffraction (XRD) measurement of AmSS>

The composition obtained in Examples was ground in a mortar, and XRD was measured by the following method. All sample preparation and measurements were performed under air atmosphere and measurement data were analyzed using HighScore Plus XRD analysis software.

Apparatus: X-ray analyzer Aeris manufactured by Malvern Panalytical
X-ray source: CuKα1 (1.540 Å), CuKα2 (1.544 Å), Kα2/Kα1 ratio = 0.5
Power: 40 kV and 7.5 mA
Geometry: Bragg-Brentano method, horizontal sample type (θ-θ)
Step size: 0.02°2θ
Scan step time: 148.9 s
Scanning range: continuous scanning from 2θ = 4° (start) to 2θ = 80° (end)

<Example 1> Production of AmSS using NaSS and ammonium sulfate (1)

A cylindrical 2 L glass separable flask equipped with a reflux condenser was charged with 290.00 g of NaSS powder, 3.17 g of 4-methoxyphenol, 185.04 g of ammonium sulfate, and 719.30 g of ion-exchanged water, and the mixture was subjected to a cation exchange reaction while being stirred at an internal temperature of 45°C for 60 minutes using a stirrer. Thereafter, the mixture was cooled to 25°C over 4 hours and aged in that state for 2 hours to obtain a white slurry liquid.

Subsequently, the slurry liquid was subjected to centrifugal filtration (600G × 15 minutes, normal temperature) to obtain 203.68 g of plate-shaped wet crystals (Fig. 3). The slurry had a good deliquoring properties, and the moisture of the wet crystal determined by an infrared moisture meter was 8.00% by weight.

The wet crystal had a sodium component of 0.27% by weight (theoretical Na component in pure NaSS was 11.1% by weight) determined by ICP, a nitrogen component of 6.7% by weight (theoretical nitrogen component in pure AmSS was 7.0% by weight) determined by elemental analysis, and a molar ratio of ammonium cations to styrenesulfonic acid units determined by $^1$H-NMR was 1.00 (Fig. 2) (The theoretical molar ratio of ammonium cations to styrenesulfonic acid units in pure AmSS was 1.00.), and thus the wet crystal was determined to be an AmSS composition as a target product.

The AmSS content, i.e. purity, of the above AmSS composition determined by redox titration was 90.6% by weight (yield based on the molar amount of raw material NaSS was 74%). The reaction formulation and results are

summarized in Table 1.

It is apparent that the alkali metal component, the halogen component, and the polymer component in the AmSS composition are smaller and higher in purity than those in Comparative Examples 1 to 2, Comparative Examples 4 to 6, Comparative Example 7, and Comparative Example 11 (Tables 5 and 7) described later.

In addition, since the median diameter of AmSS is 420 μm, which is much larger than 11 μm (Table 7) shown in Comparative Example 11, the dust generation properties can be expected to be lower than those of Comparative Example 11.

In addition, since the AmSS composition has less moisture and contains 460 ppm of 4-methoxyphenol, it is apparent that the AmSS composition has excellent storage stability as compared with Comparative Example 3 (Table 5). Furthermore, it is apparent that coloring is less likely to occur as compared with Comparative Examples 8 to 10 (Table 6) since the lithium component and nitrite component are low.

In addition, the charged charge amount per unit mass of the AmSS composition was 0.083 μC/g, which was slightly lower than 0.110 μC/g of Comparative Example 11 (Table 7) having less moisture.

[Table 1]

Table 1

| | | Example 1 | Example 2 | Example 3 | Example 4 |
|---|---|---|---|---|---|
| [Composition to be charged]<br>Sodium styrenesulfonate<br>(NaSS) | <br>9<br>mol | <br>290.00<br>1.24 | <br>290.00<br>1.24 | <br>290.00<br>1.24 | <br>290.00<br>1.24 |
| Methoxyphenol<br>(With respect to NaSS) | g<br>mol% | 3.17<br>2.06 | 7.00<br>4.55 | 3.00<br>1.95 | 1.58<br>1.03 |
| Ammonium sulfate | g mol | 185.04 1.40 | 230.00 1.74 | 160.00 1.21 | 184.55 1.40 |
| Ion-exchanged water | g | 719.30 | 750.00 | 550.00 | 1450.00 |
| AmSS seed crystal<br><br>(With respect to NaSS) | g<br>mol<br>mol% | | | | 52.73<br>0.24<br>19.08 |
| NH4/NaSS molar ratio | Equivalent | 2.26 | 2.81 | 1.95 | 2.25 |
| Total solids content | wt% | 38.19 | 39.63 | 43.08 | 25.46 |
| [Temperature conditions]<br>Heating/reaction temperature<br>Cooling/filtration temperature | <br>°C<br>°C | <br>45<br>25 | <br>45<br>25 | <br>60<br>30 | <br>45<br>10 |
| [AmSS crystal recovery conditions] | | Centrifugal filtration: room temperature x 15 minutes | | | |
| [Results]<br>AmSS wet crystal | <br>g | <br>203.68 | <br>195.36 | <br>214.30 | <br>239.12 |
| Purity | wt% | 90.6 | 90.8 | 89.9 | 88.3 |
| Yield based on the molar amount of raw material NaSS | % | 74 | 71 | 77 | 85 |
| Moisture | wt% | 8.00 | 7.86 | 8.41 | 9.89 |
| Methoxyphenol content | ppm | 460 | 889 | 356 | 156 |
| Na content | wt% | 0.27 | 0.27 | 0.45 | 0.46 |
| Li content | ppm | ≤1 | | ≤1 | ≤1 |
| Halogen content | wt% | 0.06 | 0.05 | 0.07 | 0.08 |
| Nitrite content | ppm | 15 | 13 | 16 | 16 |
| Polymer component | wt% | 0.00 | 0.00 | 0.00 | 0.00 |
| Total sulfur component | wt% | 15.0 | 15.0 | 14.8 | 14.8 |

(continued)

| | | Example 1 | Example 2 | Example 3 | Example 4 |
|---|---|---|---|---|---|
| Total nitrogen component | wt% | 6.7 | 6.7 | 6.5 | 6.5 |
| Median diameter | μm | 420 | 349 | 374 | 356 |
| [Storage stability]<br>Polymer component after storage at 60°C for 10 days | wt% | 0.00 | 0.00 | 0.01 | 0.01 |
| Polymer component after storage at 60°C for 60 days | wt% | 0.04 | 0.04 | 0.06 | 0.08 |
| APHA* after 60°C x 60 days | | 65 | 71 | 78 | 80 |
| ***AmSS** composition (as prepared) 10 wt% aqueous solution | | | | | |

<Example 2> Production of AmSS using NaSS and ammonium sulfate (2)

A cylindrical 2 L glass separable flask equipped with a reflux condenser was charged with 290.00 g of NaSS powder, 7.00 g of 4-methoxyphenol, 230.00 g of ammonium sulfate, and 750.00 g of ion-exchanged water, and the mixture was subjected to a cation exchange reaction while being stirred at an internal temperature of 45°C for 60 minutes using a stirrer. Thereafter, the mixture was cooled to 35°C over 30 minutes and held for 10 minutes, and then the internal temperature was raised to 45°C again. When the internal temperature reached 45°C, the heating was stopped, the mixture was cooled to 25°C over 4 hours, and aged in that state for 2 hours to obtain a white slurry liquid.

Subsequently, the slurry liquid was subjected to centrifugal filtration under the same conditions as in Example 1 to obtain 195.36 g of plate-shaped wet crystals. The deliquoring properties of the slurry was good, and the moisture determined by an infrared moisture meter were 7.86% by weight.

The wet crystal had a sodium component of 0.27% by weight (theoretical Na component in pure NaSS was 11.1% by weight) determined by ICP, a nitrogen component of 6.7% by weight (theoretical nitrogen component in pure AmSS was 7.0% by weight) determined by elemental analysis, and a molar ratio of ammonium cations to styrenesulfonic acid units determined by $^{1}$H-NMR was 1.01 (the theoretical molar ratio of ammonium cations to styrenesulfonic acid units in pure AmSS was 1.00), and thus the wet crystal was determined to be an AmSS composition as a target product.

The AmSS content of the above AmSS composition determined by redox titration, that is, the purity was 90.8% by weight (yield based on the molar amount of raw material NaSS: 71%). The reaction formulation and results are summarized in Table 1. It is apparent that the alkali metal component, the halogen component, and the polymer component in the AmSS composition are smaller and higher in purity than those in Comparative Examples 1 to 2, Comparative Examples 4 to 6, Comparative Example 7, and Comparative Example 11 (Tables 5 and 7).

In addition, since the median diameter of AmSS is 349 μm, which is much larger than 11 μm (Table 7) shown in Comparative Example 11, the dust generation properties can be expected to be lower than that of Comparative Example 11.

In addition, since the AmSS composition has less moisture and contains 889 ppm of 4-methoxyphenol, it is apparent that the AmSS composition has excellent storage stability as compared with Comparative Example 3 (Table 5). Furthermore, it is apparent that coloring is less likely to occur as compared with Comparative Examples 8 to 10 (Table 6) since the lithium component and nitrite component are low.

In addition, the charged charge amount per unit mass of the AmSS composition was 0.082 μC/g, which was slightly lower than that of Comparative Example 11 (Table 7) having less moisture.

<Example 3> Production of AmSS using NaSS and ammonium sulfate (3)

-Synthesis of AmSS-

In Example 1, the composition to be charged and temperature conditions were changed to the conditions shown in Table 1, and plate-shaped wet crystals presumed to be AmSS were obtained by the same operations as in Example 1.

From the analysis results shown in Table 1, the wet crystals were determined to have the AmSS composition. It is apparent that the alkali metal component, the halogen component, and the polymer component in the AmSS composition are smaller and higher in purity than those in Comparative Examples 1 to 2, Comparative Examples 4 to 6, Comparative Example 7, and Comparative Example 11 (Tables 5 and 7).

In addition, since the median diameter of AmSS is 374 μm, which is much larger than 11 μm (Table 7) shown in Comparative Example 11, the dust generation properties can be expected to be lower than that of Comparative Example

11.

In addition, since the AmSS composition contains 356 ppm of 4-methoxyphenol, it is apparent that the AmSS composition has excellent storage stability as compared with Comparative Example 3 (Table 5). Furthermore, it is apparent that coloring is less likely to occur as compared with Comparative Examples 8 to 10 (Table 6) since the lithium component and nitrite component are low.

In addition, the charged charge amount per unit mass of the AmSS composition was 0.082 $\mu$C/g, which was slightly lower than that of Comparative Example 11 (Table 7) having less moisture.

-Polymerization of AmSS-

The radical polymerizability of the AmSS composition was confirmed.

In a 300 ml three-necked flask reactor equipped with a cooling tube, a magnetic induction type stirrer, and a nitrogen introduction tube, 10.00 g of the AmSS wet crystals obtained above, 1.00 g of an aqueous polymerization initiator solution (1.85% by weight aqueous solution of V -50), and 80.00 g of ion-exchanged water were collected, and deoxygenated by repeating decompression with an aspirator and nitrogen introduction. Thereafter, the reactor was immersed in a warm bath at 60°C, and polymerization was initiated while stirring. Sampling was performed at regular time intervals, and the polymerization conversion and the molecular weight were measured by GPC. From the results shown in Table 2, it is apparent that there is no significant difference in polymerization rate and molecular weight as compared with Examples 2 and 4 and Comparative Example 3 containing less methoxyphenol.

[Table 2]

Table 2

| | | Example 2 | Example 3 | Example 4 | Comparative Example 3 | Comparative Example 6 |
|---|---|---|---|---|---|---|
| [Composition to be charged] | | | | | | |
| AmSS composition(as prepared) | 9 | 10.00 | 10.00 | 10.00 | 10.00 | 10.00 |
| Moisture | wt% | 7.86 | 8.41 | 9.89 | 7.54 | 7.75 |
| Methoxyphenol content | ppm | 889 | 356 | 156 | 16 | 2204 |
| 1 85% by weight V-50 aqueous solution | g | 1.00 | 1.00 | 1.00 | 1.00 | 1.00 |
| Ion-exchanged water | g | 80.00 | 80.00 | 80.00 | 80.00 | 80.00 |
| [Polymerization temperature] | | 60°C | | | | |
| [Polymerization conversion rate] | | | | | | |
| 0.5h | % | 41.5 | 43.1 | 42.8 | 44.2 | 24.5 |
| 1.0h | % | 64.6 | 66.2 | 65.4 | 67.8 | 44.6 |
| 2.0h | % | 82.8 | 84.4 | 81.7 | 85.1 | 79.3 |
| 5.0h | % | 96.8 | 96.6 | 97.4 | 97.3 | 90.9 |
| [Number average molecular weight] | | | | | | |
| 0.5h | | 334K | 334K | 334K | 338K | 275K |
| 1.0h | | 329K | 324K | 324K | 329K | 270K |
| 2.0h | | 315K | 320K | 320K | 324K | 270K |
| 5.0h | | 315K | 324K | 320K | 324K | 260K |

<Example 4> Production of AmSS using NaSS and ammonium sulfate (4)

-Synthesis of AmSS-

A cylindrical 2 L glass separable flask equipped with a reflux condenser was charged with 290.00 g of NaSS powder, 1.58 g of 4-methoxyphenol, 184.55 g of ammonium sulfate, and 1450.00 g of ion-exchanged water, and the mixture was subjected to a cation exchange reaction while being stirred at an internal temperature of 45°C for 30 minutes using a stirrer. Thereafter, the mixture was cooled to an internal temperature of 30°C over 30 minutes to form a slurry, then 52.73 g of the AmSS composition obtained in Example 1 was added as seed crystals, and the mixture was stirred as it was for 10 minutes (charging ratio of ammonium cation derived from ammonium sulfate to NaSS was 2.25 equivalent, and total solids content was 25.46% by weight). Thereafter, the mixture was cooled to 10°C over 3 hours and aged in that state for 2 hours to obtain a white slurry liquid. Thereafter, the slurry liquid was subjected to centrifugal filtration in the same manner as in Example 1 to obtain 239.12 g of plate-shaped wet crystals.

From the analysis results shown in Table 1, the wet crystals were determined to have the AmSS composition. Since the AmSS composition contains 156 ppm of 4-methoxyphenol, it is apparent that the AmSS composition has excellent storage stability as compared with Comparative Example 3 (Table 5). Furthermore, it is apparent that coloring is less likely to occur as compared with Comparative Examples 8 to 10 (Table 6) since the lithium component and nitrite component are low.

In addition, since the median diameter of AmSS is 356 μm, which is much larger than 11 μm (Table 7) shown in Comparative Example 11, the dust generation properties can be expected to be lower than that of Comparative Example 11. In addition, the charged charge amount per unit mass of the AmSS composition was 0.077 μC/g, which was slightly lower than that of Comparative Example 11 (Table 7) having less moisture.

<Polymerization of AmSS Composition>

The radical polymerizability of the AmSS composition was confirmed under the same conditions as in Example 3. From the results shown in Table 2, it is apparent that there is no significant difference in polymerization rate and molecular weight as compared with Examples 2 and 3 and Comparative Example 3 containing less methoxyphenol.

<Example 5> Production of AmSS using NaSS and ammonium sulfate (5)

A cylindrical 2 L glass separable flask equipped with a reflux condenser was charged with 423.79 g of NaSS powder, 4.65 g of 4-methoxyphenol, 269.90 g of ammonium sulfate, and 1048.89 g of ion-exchanged water, and the mixture was subjected to a cation exchange reaction while being stirred at an internal temperature of 45°C for 60 minutes using a stirrer. Thereafter, the mixture was cooled to 15°C over 5 hours and aged in that state for 2 hours to obtain a white slurry liquid.

Subsequently, the slurry liquid was subjected to centrifugal filtration (600G × 20 minutes, normal temperature) to obtain 320.66 g of plate-shaped wet crystals. The slurry had a good deliquoring properties, and the moisture of the wet crystal determined by an infrared moisture meter was 5.10% by weight.

The wet crystals were dried at 40°C for 30 minutes using a rotary evaporator (pressure: 660 Pa) to obtain 293.35 g of dried crystals.

Since the sodium component determined by ICP of AmSS was 0.21% by weight, the nitrogen component determined by elemental analysis was 6.8% by weight, and the molar ratio of the ammonium cation to the styrenesulfonic acid unit determined by $^{1}$H-NMR was 1.00, the dried crystals were determined to be an AmSS composition as a target product.

The AmSS content of the dried crystals, determined by redox titration, i.e., purity, was 98.4% by weight (yield based on the molar amount of raw material NaSS: 82%). The reaction formulation and results are summarized in Table 3.

The wet crystals had good drying properties, and the moisture after drying by a rotary evaporator was reduced to 0.27% by weight. These drying properties did not change even after the wet crystals were stored in a sealed container at least at room temperature for 6 months. On the other hand, NaSS used as a raw material had a moisture of 3.69% by weight even when dried under the same conditions. That is, it has been found that the moisture contained in the wet crystal of AmSS is attached water, and the drying properties are completely different from that of the crystal water contained in the raw material NaSS. That is, it is considered that the AmSS composition of the present invention is stabilized by anhydrous salt crystals.

It is apparent that the alkali metal component, the halogen component, and the polymer component in the AmSS composition are smaller and higher in purity than those in Comparative Examples 1 to 2, Comparative Examples 4 to 6, Comparative Example 7, and Comparative Example 11 (Tables 5 and 7) described later.

In addition, since the median diameter of AmSS is 367 μm, which is much larger than 11 μm (Table 7) shown in Comparative Example 11, the dust generation properties can be expected to be lower than that of Comparative Example 11.

In addition, since the AmSS composition has less moisture and contains 289 ppm of 4-methoxyphenol, it is apparent that the AmSS composition has excellent storage stability as compared with Comparative Example 3 (Table 4). Furthermore, it is apparent that coloring is much less likely to occur as compared with Comparative Examples 8 to 10 (Table 6) since the lithium component and nitrite component are low. Incidentally, the polymer component after extending the storage stability test at 60°C to at least 210 days was 0.03% by weight, and thus extremely high stability was exhibited.
The AmSS composition had a charge amount per unit mass of 0.093 μC/g, which was higher than that in Examples 1 to 4, but slightly lower than that in Comparative Example 11 (Table 7) having a small particle size.
In addition, as a result of comparing the PXRD patterns of the wet AmSS, the dried AmSS, and the NaSS used as a raw material obtained in the Examples (Figs. 7 to 12), the diffraction patterns of the AmSS and the NaSS were clearly different. This is largely due to the difference between NaSS stabilized with hemihydrate salt crystals and AmSS stabilized with anhydrous salt crystals.

[Table 3]

Table 3

| | | Example 5 |
|---|---|---|
| [Composition to be charged]<br>Sodium styrenesulfonate<br>(NaSS) | <br>g<br>mol | <br>423.79<br>1.81 |
| Methoxyphenol<br>(With respect to NaSS) | g<br>mol% | 4.65<br>2.07 |
| Ammonium sulfate | g<br>mol | 269.90<br>2.04 |
| Ion-exchanged water | g | 1048.89 |
| Total solids content | wt% | 38.22 |
| [Temperature conditions]<br>Heating/reaction temperature<br>Cooling/filtration temperature | <br>°C<br>°C | <br>45<br>15 |
| [AmSS crystal recovery conditions] | | Centrifugal filtration room temperaturex20 minutes |
| [AmSS crystal drying conditions] | | Rotary evaporator 40°C×30 minutes at 660Pa |
| [Results]<br>AmSS composition(dried crystal) | <br>9 | <br>293.35 |
| Purity | wt% | 98.4 |
| Yield based on the molar amount of raw material NaSS | % | 82 |
| Moisture | wt% | 0.27 |
| Methoxyphenol content | ppm | 289 |
| Na content | wt% | 0.21 |
| Li content | ppm | ≤1 |
| Haloen content | wt% | 0.06 |
| Nitrite content | ppm | 19 |
| Polymer component | wt% | 0.00 |
| Total sulfur component | wt% | 15.3 |
| Total nitrogen component | wt% | 6.8 |
| Median diameter | μm | 367 |
| [Storage stability] | | |

(continued)

| | | Example 5 |
|---|---|---|
| Polymer component after storage at 60°C for 10 days | wt% | 0.01 |
| Polymer component after storage at 60°C for 60 days | wt% | 0.01 |
| APHA* after 60°C x 60 days | - | 60 |
| *AmSS composition (as prepared) 10 wt% aqueous solution | | |

<Example 6> Purification of AmSS composition

Into a cylindrical 0.5 L glass separable flask equipped with a reflux condenser were charged 132.90 g of the AmSS composition obtained in Example 1, 0.74 g of 4-methoxyphenol, and 85.00 g of ion-exchanged water, and the mixture was stirred at an internal temperature of 55°C for 60 minutes using a stirrer. Thereafter, the mixture was cooled to an internal temperature of 35°C over 60 minutes, and then heated again to 45°C. When the internal temperature reached 45°C, the heating was stopped, and the internal temperature was cooled to 25°C over 4 hours. The mixture was aged as it was for 2 hours, and then the slurry liquid was subjected to centrifugal filtration in the same manner as in Example 1, thereby obtaining 95.43 g of an AmSS composition.

As shown in Table 4, it is apparent that the purity is higher as compared to Examples 1 to 4, and the storage stability is excellent as compared to Comparative Example 3 (Table 5). Furthermore, it is apparent that coloring is much less likely to occur as compared with Comparative Examples 8 to 10 (Table 6) since the lithium component and nitrite component are low.

In addition, since the median diameter is 278 μm, which is much larger than 11 μm (Table 7) shown in Comparative Example 11, it is apparent that the dust generation properties are low.

Further, as a result of drying the AmSS composition at 40°C for 30 minutes (pressure: 665 Pa) using a rotary evaporator, the drying properties were good, the moisture decreased to 0.31wt%, and the purity increased to 99.3%.

The dry AmSS composition was collected in a glass petri dish and allowed to stand for 24 hours in a thermo-hygrostat at 30°C and a relative humidity of 75%, and the moisture was 0.52% by weight. On the other hand, as a result of performing the same operation with NaSS having a moisture of 0.15% by weight prepared by vacuum drying at 60°C for 12 hours, the moisture increased to 4.95% by weight. From this, it is considered that NaSS is stabilized by hemihydrate salt crystals, whereas the AmSS composition of the present invention is stabilized by anhydrous salt crystals.

The dried AmSS composition was crushed at normal temperature and 4000 rpm for 10 seconds using a small desktop power mill (P-02S manufactured by SHOWA KAGAKUKIKAI CO.,LTD), and as a result, the median diameter decreased to 56 μm. In the same manner as described above, the crushed product was collected in a glass petri dish and allowed to stand for 24 hours in a thermo-hygrostat at 30°C and a relative humidity of 75%. As a result, although the median diameter largely decreased, the moisture increased only up to 0.58% by weight. This is because the AmSS composition is stabilized by anhydrous salt crystals.

[Table 4]

Table 4

| | | Example 6 |
|---|---|---|
| [Composition to be charged] | | |
| Ammonium styrenesulfonate | g | 132.90 |
| (AmSS) | mol | 0.60 |
| Moisture | wt% | 8.00 |
| Purity | wt% | 90.60 |
| Methoxyphenol | g | 0.74 |
| (With respect to AmSS) | mol% | 1.00 |
| Ion-exchanged water | g | 85.00 |
| Total solids content | wt% | 56.26 |
| [Temperature conditions] | | |

(continued)

| | | Example 6 |
|---|---|---|
| Heating temperature | °C | 55 |
| Cooling/filtration temperature | °C | 25 |
| [AmSS crystal recovery conditions] Centrifugal filtration: room temperature x 15 minutes | | |
| [Results] | | |
| AmSS wet crystal | g | 95.43 |
| Purity | wt% | 956 |
| Yield (Calculated as pure compound) | % | 76 |
| Moisture | wt% | 4.14 |
| Methoxyphenol content | | 474 |
| Na content | wt% | 0.03 |
| Li content | ppm | ≤1 |
| Halogen content | wt% | 0.01 |
| Nitrite component | ppm | 6 |
| Polymer component | wt% | 0.00 |
| Total sulfur component | wt% | 15.3 |
| Total nitrogen component | wt% | 6.7 |
| Median diameter | μm | 278 |
| [Storage stability] | | |
| Polymer component after storage at 60°C for 10 days | wt% | 0.00 |
| Polymer component after storage at 60°C for 60 days | wt% | 0.04 |
| APHA* after 60°C x 60 days | | 62 |
| *AmSS composition (as prepared) 10 wt% aqueous solution | | |

<Comparative Example 1> Production of AmSS using NaSS and ammonium sulfate (6)

An attempt was made to produce AmSS by changing the composition to be charged and the temperature conditions to the conditions shown in Table 5 in Example 4. From the analysis results of the sodium and nitrogen contents in the obtained wet crystals (Table 5), the wet crystals were determined to have the AmSS composition, but since the slurry had a poor deliquoring properties, the moisture of the composition was as high as 18.90% by weight and the purity was as low as 77.2%, and the purity was obviously inferior to that of Examples 1 to 4.

Although 169 ppm of 4-methoxyphenol was present, the storage stability was clearly inferior to that of Examples 1 to 4. The reason for the increase in moisture is considered to be that the total solids content during the reaction was too low.

<Comparative Example 2> Production of AmSS using NaSS and ammonium sulfate (7)

An attempt was made to produce AmSS by changing the composition to be charged and the temperature conditions to the conditions shown in Table 5 in Example 4. From the analysis results of the sodium and nitrogen contents in the obtained wet crystals (Table 5), the wet crystals were determined to have the AmSS composition, but since the slurry had a poor deliquoring properties, the moisture of the composition was as high as 21.00% by weight and the purity was as low as 75.2%, and the purity was obviously inferior to that of Examples 1 to 4.

Although 174 ppm of 4-methoxyphenol was present, the storage stability was clearly inferior to that of Examples 1 to 4. It is considered that this is because the moisture was high, and the reason why the moisture was increased is because the amount of ammonium sulfate added to NaSS during the reaction was too large.

<Comparative Example 3> Production of AmSS using NaSS and ammonium chloride (1)

An attempt was made to produce AmSS by changing the inorganic ammonium salt species, the composition to be charged, and the temperature conditions to those shown in Table 5 in Example 4. From the analysis results of the sodium and nitrogen contents in the obtained wet crystals (Table 5), the wet crystals were determined to have the AmSS composition. The slurry had a good deliquoring properties, the moisture of the AmSS composition was as low as 7.54% by weight, and the halogen content was slightly high, but the purity was 90.1%, which was equivalent to Examples 1 to 4.
However, the storage stability was clearly inferior to that of Examples 1 to 4. This is considered to be because the moisture was low but the 4-methoxyphenol content was too low at 16 ppm.

<Comparative Example 4> Production of AmSS using NaSS and ammonium chloride (2)

An attempt was made to produce AmSS by changing the inorganic ammonium salt species, the composition to be charged, and the temperature conditions to those shown in Table 5 in Example 1. From the analysis results of the sodium and nitrogen contents in the obtained wet crystals (Table 5), the wet crystals were determined to have the AmSS composition, but since the slurry had poor deliquoring properties, the moisture of the composition was 17.30% by weight, the halogen content was as high as 2.01% by weight, and the purity was as low as 80.1%, and the purity was clearly inferior to that of Examples 1 to 4. This is considered to be because the reaction temperature was too low.
Although the 4-methoxyphenol content in the AmSS composition was 78 ppm, the storage stability was significantly inferior to that of Examples 1 to 4. This is considered to be because there was a lot of moisture.

<Comparative Example 5> Production of AmSS using NaSS and ammonium chloride (3)

An attempt was made to produce AmSS by changing the inorganic ammonium salt species, the composition to be charged, and the temperature conditions to those shown in Table 5 in Example 1. As a result of analyzing the obtained wet crystals, it was found that the sodium content was as high as 6.47 wt%, and the cation exchange rate was less than 50% (Table 5). It is considered that the addition molar ratio of the ammonium cation to NaSS was too low, as low as 0.96 equivalents.

<Comparative Example 6> Production of AmSS using NaSS and ammonium sulfate (8)

-Synthesis of AmSS-

An attempt was made to produce AmSS by changing the composition to be charged and the temperature conditions to the conditions shown in Table 5 in Example 4. From the results of the sodium content and nitrogen content in the obtained wet crystals, the wet crystals were determined to have the AmSS composition (Table 5). The slurry had a good deliquoring properties, and the AmSS composition had a low moisture of 7.75% by weight and a low halogen content of 0.02% by weight and a high purity of 90.4%, which were equivalent to those in Example 4.
However, since the amount of 4-methoxyphenol added during the reaction was increased, the 4-methoxyphenol content in the AmSS composition was increased to 2204 ppm. Therefore, the spontaneous polymerizability at the time of storage at 60°C was suppressed to the same level as in Examples 1 to 3, but the APHA value increased, and further, as shown in Table 2 and the following, the polymerizability at the time of producing the polymer was adversely affected.

-Polymerization of AmSS-

The polymerizability of the AmSS composition was confirmed under the same conditions as in Example 3. As a result, it is apparent that the polymerization rate is clearly slower and the molecular weight is lower than those in Examples 1 to 4 and Comparative Example 3 in which the amount of 4-methoxyphenol is low (Table 2).

<Comparative Example 7> Production of AmSS using NaSS and ammonium sulfate (9)

An attempt was made to produce AmSS by changing the reaction temperature from 60°C to 85°C in Example 3. From the analysis results of the sodium content and nitrogen content of the obtained wet crystals, the wet crystals were determined to have the AmSS composition (Table 5). However, since the deliquoring properties of the slurry were poor and the moisture of the AmSS composition was as high as 13.70% by weight, the purity was as low as 84.3% by weight and was clearly inferior to that of Examples 1 to 4. It is considered that since the reaction temperature was too high, high-quality crystals were not generated, and the amount of polymer produced increased, leading to deterioration of the deliquoring properties.

In addition, although the content of 4-methoxyphenol in the AmSS composition was 394 ppm, the AmSS composition had a large amount of moisture, and thus the AmSS composition was significantly inferior in storage stability to Examples 1 to 4.

[Table 5]

Table 5

| | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| [Composition to be charged] | | | | | | | | |
| Sodium styrenesulfonate (NaSS) | g | 110.00 | 110.00 | 110.00 | 205.00 | 205.00 | 290.00 | 290.00 |
| | mol | 0.47 | 0.47 | 0.47 | 0.88 | 0.88 | 1.24 | 1.24 |
| Methoxyphenol | g | 0.60 | 0.60 | 0.30 | 0.30 | 1.10 | 15.00 | 3.00 |
| (With respect to NaSS) | mol% | 1.03 | 1.03 | 0.51 | 0.28 | 1.02 | 9.74 | 1.95 |
| Ammonium sulfate | g | 70.00 | 100.00 | | | | 180.00 | 160.00 |
| | mol | 0.53 | 0.76 | | | | 1.36 | 1.21 |
| Ammonium chloride | g | | | 55.68 | 130.00 | 45.00 | | |
| | mol | | | 1.04 | 2.43 | 0.84 | | |
| Ion-exchanged water | g | 650.00 | 50000 | 400.00 | 700.00 | 415.00 | 75000 | 550.00 |
| AmSS seed crystal (90.6%) | g | 20.00 | 20.00 | 20.00 | | | 20.00 | |
| | mol | 0.09 | 0.09 | 0.09 | | | 0.09 | |
| (With respect to NaSS) | mol% | 19.08 | 19.08 | 19.08 | | | 7.24 | |
| NH4/NaSS molar ratio | Equivalent | 2.25 | 3.22 | 2.21 | 2.77 | 096 | 2.20 | 1.95 |
| Total solids content | wt% | 22.47 | 30.27 | 30.12 | 30.96 | 35.48 | 39.07 | 43.08 |
| [Temperature conditions] | | | | | | | | |
| Heating/reaction temperature | °C | 60 | 60 | 60 | 25 | 60 | 45 | 85 |
| Cooling/filtration temperature | °C | 10 | 15 | 25 | 25 | 25 | 25 | 30 |
| [AmSS crystal recovery conditions] | | Centrifugal filtration: room temperature x 15 minutes | | | | | | |
| [Results] | | | | | | | | |
| AmSS wet crystal | g | 88.40 | 96.70 | 84.30 | 160.87 | 200.10 | 211.40 | 235.40 |
| Purity | wt% | 77.2 | 75.2 | 90.1 | 80.1 | 70.9 | 90.4 | 84.3 |
| Yield based on the molar % amount of raw material NaSS | | 72 | 77 | 80 | 73 | 80 | 77 | 80 |
| Moisture | wt% | 18.90 | 21.00 | 7.54 | 17.30 | 18.70 | 7.75 | 13.70 |

(continued)

| | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| Methoxypheno content | ppm | 169 | 174 | | 78 | Not measured | 2204 | 394 |
| Na content | wt% | 0.69 | 0.75 | 0.35 | 0.53 | 6.47 | 0.37 | 0.51 |
| Li content | ppm | ≤1 | ≤1 | ≤1 | ≤1 | Not measured | <1 | ≤1 |
| Halogen content | wt% | 0.13 | 0.14 | 0.63 | 2.01 | Not measured | 0.02 | 0.13 |
| Nitrite content | ppm | 26 | 25 | 15 | 29 | Not measured | | 23 |
| Polymer component | wt% | 0.00 | 0.00 | 0.00 | 0.00 | Not measured | 0.00 | 0.15 |
| Total sulfur component | wt% | 12.9 | 12.6 | 14.7 | 13.2 | Not measured | 14.7 | 13.8 |
| Total nitrogen component | wt% | 5.6 | 5.5 | 6.4 | 5.8 | Not measured | 6.4 | 6.0 |
| Median diameter | μm | 204 | 174 | 450 | 158 | Not measured | 512 | 374 |
| [Storage stability] | | | | | | | | |
| Polymer component after storage at 60°C for 10 days | wt% | 0.32 | 0.51 | 0.15 | 0.58 | Not measured | 0.00 | 0.22 |
| Polymer component after storage at 60°C for 60 days | wt% | 2.42 | 2.63 | 0.53 | 3.01 | Not measured | 0.01 | 1.03 |
| APHA* after 60°C x 60 days | - | 230 | 240 | 260 | 238 | Not measured | 103 | 260 |
| *AmSS composition (as prepared) 10 wt% aqueous solution | | | | | | | | |

<Comparative Example 8> Production of AmSS using LiSS and ammonium chloride (1)

A cylindrical 2 L glass separable flask equipped with a reflux condenser was charged with 541.54 g of LiSS powder, 1.20 g of lithium nitrite (40% by weight aqueous solution), 150.18 g of ammonium chloride, and 1135.09 g of ion-exchanged water, and the mixture was subjected to a cation exchange reaction while being stirred at an internal temperature of 35°C for 60 minutes using a stirrer. Thereafter, the mixture was cooled to 5°C over 5 hours and cooled to an internal temperature of 5°C. The mixture was aged in that state for 2 hours to obtain a white slurry liquid. Subsequently, the slurry liquid was subjected to centrifugal filtration under the same conditions as in Example 1 to obtain 423.65 g of rhombic plate-shaped wet crystals. The deliquoring properties of the slurry were good, and the moisture determined by an infrared moisture meter was 9.62% by weight.

The lithium component of the wet crystal determined by ICP was 0.13% by weight (theoretical Li content in pure LiSS was 3.65% by weight), the sodium component was 5 ppm, the nitrogen component of the AmSS composition determined by elemental analysis was 6.3% by weight (theoretical nitrogen component in pure AmSS was 7.0% by weight), and the molar ratio of the ammonium cation to the styrenesulfonic acid unit determined by $^1$H-NMR was 1.01(the theoretical molar ratio of ammonium cations to styrenesulfonic acid units in pure AmSS is 1.00), so that the wet crystal was determined to be an AmSS composition as a target product.

The AmSS content of the above AmSS composition determined by redox titration, that is, the purity was 89.2% (yield based on raw material LiSS mole: 77%). As summarized in Table 6, the purity of the AmSS composition was equivalent to that in Examples 1 to 4 (Table 1), but since 4-methoxyphenol was not contained, the AmSS composition was significantly inferior in storage stability to Examples 1 to 4.

<Comparative Example 9> Production of AmSS using LiSS and ammonium sulfate (2)

AmSS production was carried out by changing the inorganic ammonium salt species, the composition to be charged, and the temperature conditions to those shown in Table 6, and adding 4-methoxyphenol in Comparative Example 9. From the analysis result of the lithium content of the wet crystals, the wet crystals were determined to have the AmSS composition. The slurry had good deliquoring properties, and the AmSS composition had a low moisture of 6.82% by weight and a low halogen content of 0.09% by weight and a high purity of 92.5%, which were equivalent to those of Examples 1 to 4.

The content of 4-methoxyphenol in the AmSS composition was 805 ppm, and the spontaneous polymerizability at the time of storage at 60°C was suppressed to the same level as in Examples 1 to 4, but the APHA value increased. The reason is not clear, but it is presumed that some interaction between a lithium cation and 4-methoxyphenol is involved.

<Comparative Example 10> Production of AmSS using LiSS and ammonium sulfate (3)

AmSS was produced by changing the type and addition amount of the polymerization inhibitor to the conditions shown in Table 6 in Comparative Example 10. From the analysis result of the lithium content of the wet crystals, the wet crystals were determined to have the AmSS composition. The slurry had good deliquoring properties, and the AmSS composition had a low moisture of 6.91% by weight and a low halogen content of 0.08% by weight and a high purity of 92.6%, which were equivalent to those of Examples 1 to 4.

The content of methoxyphenol in the AmSS composition was 420 ppm, the content of nitrite was 72 ppm, and the spontaneous polymerizability at the time of storage at 60°C was suppressed to the same level as in Examples 1 to 4, but the APHA value increased. The reason is not clear, but it is presumed that some interaction with a lithium cation, 4-methoxyphenol, and a nitrite anion is involved.

[Table 6]

Table 6

| | | Comparative Example 8 | Comparative Example 9 | Comparative Example 10 |
|---|---|---|---|---|
| [Composition to be charged] | | | | |
| Lithium styrenesulfonate | g | 541.54 | 433.50 | 430.00 |
| (LiSS) | mol | 2.43 | 1.94 | 1.93 |
| Methoxhenol | g | | 9.51 | 5.00 |

(continued)

| | | Comparative Example 8 | Comparative Example 9 | Comparative Example 10 |
|---|---|---|---|---|
| Lithium nitrite<br>(With respect to LiSS) | g<br>mol% | 1.20<br>0.37 | <br>3.94 | 1.20<br>2.56 |
| Ammonium sulfate | g<br>mol | | 283.12<br>2.14 | 283.05<br>2.14 |
| Ammonium chloride | g<br>mol | 150.18<br>2.81 | | |
| Ion-exchanged | | 1135.09 | 1100.17 | |
| NH4/LiSS ratio | Equivalent | 1.16 | 2.20 | 2.22 |
| Total solids content | wt% | 35.76 | 38.07 | 38.02 |
| [Temperature conditions]<br>Heating/reaction temperature<br>Cooling/filtration temperature | <br>°C<br>°C | <br>35<br>5 | <br>46<br>25 | <br>45<br>25 |
| [AmSS crystal recovery conditions] | | Centrifugal filtration: room temperature x 15 minutes | | |
| [Results]<br>AmSS wet crystal | <br>g | <br>423.65 | <br>327.65 | <br>327.65 |
| Purity | wt% | 89.2 | 92.5 | 92.6 |
| Yield based on the molar amount of raw material LiSS | % | 77 | 77 | 78 |
| Moisture | wt% | 9.62 | 6.82 | 6.91 |
| Methoxyphenol content | ppm | 0 | 805 | 420 |
| Na content | ppm | 5 | 3 | 3 |
| Li content | wt% | 0.13 | 0.10 | 0.11 |
| Halogen content | wt% | 0.65 | 0.09 | 0.08 |
| Nitrite content | ppm | 88 | 16 | 72 |
| Polymer component | wt% | 0.00 | 0.00 | 0.00 |
| Total sulfur component | wt% | 14.4 | 14.8 | 14.8 |
| Total nitrogen component | wt% | 6.3 | 6.5 | 6.5 |
| Median diameter | μm | 448 | 236 | 257 |
| [Storage stability]<br>Polymer component after storage at 60°C for 10 days<br>Polymer component after storage at 60°C for 60 days | <br>wt%<br>wt% | <br>0.68<br>4.00 | <br>0.00<br>0.07 | <br>0.00<br>0.06 |
| APHA* after 60°C x 60 days | - | 330 | 198 | 365 |
| *AmSS composition (as prepared) 10 wt% aqueous solution | | | | |

<Comparative Example 11> Example 1 of JP 50-149642 A

In a 1 L four-necked flask equipped with a cooling tube, 500.00 g of methanol, 25.01 g of NaSS powder, and 25.02 g of ammonium sulfate were collected, and heated in a bath at 65°C for 3 hours while being stirred with a magnetic stirrer. The inside of the system was slightly cloudy, and the raw material was almost dissolved (charging ratio of ammonium cation to NaSS was 3.54 equivalent, total solids content was 8.79% by weight). Thereafter, the mixture was allowed to cool to 30°C, and a solid thought to be sodium sulfate was precipitated. The reaction solution was subjected to suction filtration

using a circulating aspirator to separate the precipitate, and then the filtrate was concentrated and dried using a rotary evaporator at 50°C for 2 hours to obtain 21.53 g of dried powder (Fig. 6). The moisture was 0.50% by weight.

Subsequently, the active vinyl group of the dried powder was quantified by redox titration. As a result, assuming that the dried powder was AmSS, the purity was 98.0%. However, since the sodium component determined by ICP was 5.3% by weight (theoretical Na component in pure NaSS was 11.1% by weight), the nitrogen component determined by elemental analysis was 3.4% by weight (theoretical nitrogen component in pure AmSS was 7.0% by weight), and the molar ratio of the ammonium cation to the styrenesulfonic acid unit determined by $^1$H-NMR was 0.54 (the theoretical molar ratio of the ammonium cation to the styrene sulfonic acid unit in pure AmSS was 1.00 (Fig. 5)), about 50% of the dried powder was NaSS, and the cation exchange rate was estimated to be about 50%.

The reason why the cation exchange rate was low although 3.54 equivalents of excess ammonium cation were added to the raw material NaSS is considered to be that the ionic dissociation degree of each salt in methanol was insufficient and the difference in solubility of each salt in methanol was not so large. In this method, even if the impurity NaBr in NaSS is converted into ammonium bromide by cation exchange, bromine cannot be removed because ammonium bromide is dissolved in methanol. In fact, it was found that the halogen component in the dried powder was 1.27% by weight, which was 15 times or more higher than that in Examples 1 to 4.

Although the dried powder had a low amount of moisture, the dried powder did not contain 4-methoxyphenol, and therefore was inferior in storage stability to Examples 1 to 4.

In addition, the median diameter of the dried powder was as very small as 11 μm, and the dust generation properties were clearly stronger than that of Examples 1 to 4.

In addition, since the charged charge amount per unit mass of the dried powder is 0.110 μC/g, which is larger than that in Examples 1 to 5, it is considered that the scattering properties are high and the risk of dust explosibility is also high.

[Table 7]

Table 7

| | | Comparative Example 11 |
|---|---|---|
| [Composition to be charged]<br>Sodium styrenesulfonate<br>(NaSS) mol | <br>g | <br>25.01<br>0.11 |
| Methoxyphenol<br>(With respect to NaSS) | 9 | 0.00<br>0.00 |
| Ammonium sulfate | g<br>mol | 25.02<br>0.19 |
| Methanol | | 500.00 |
| NH4/NaSS | Equivalent | 3.54 |
| Total solids content | wt% | 8.78 |
| [Temperature conditions]<br>Heating temperature<br>Cooling (filtration) temperature | <br>°C<br>°C | <br>65<br>30 |
| [AmSS crystal recovery conditions]<br>Rotary evaporator 50°C x 2 hours | | |
| [Results]<br>AmSS | | <br>21.53 |
| Purity | wt% | 98.0 |
| Yield based on the molar amount of raw material NaSS | % | 98 |
| Moisture | wt% | 0.50 |
| Methoxyphenol content | ppm | 0 |
| Na content | wt% | 5.30 |

(continued)

| | | Comparative Example 11 |
|---|---|---|
| Li content | ppm | ≤1 |
| Halogen content | wt% | 1.27 |
| Nitrite content | ppm | 65 |
| Polymer component | wt% | 0.02 |
| Total sulfur component | wt% | 15.0 |
| Total nitrogen component | wt% | 3.4 |
| Median diameter | μm | 11 |
| APHA value* | | 60 |
| [Storage stability] | | |
| Polymer component after storage at 60°C for 10 days | wt% | 0.04 |
| Polymer component after storage at 60°C for 60 days Polymer component after storage at 60°C for 60 days wt% | wt% | 0.23 |
| APHA* after 60°C x 60 days | - | 125 |
| *AmSS composition (as prepared) 10 wt% aqueous solution | | |

<Example 7> Production of AmSS using NaSS and ammonium sulfate (10) ETPE

Into a cylindrical 0.5 L glass separable flask equipped with a reflux condenser were charged 73.01 g of ammonium sulfate and 255.00 g of ion-exchanged water, which were dissolved while being heated to an internal temperature of 40°C under stirring. After the dissolution of ammonium sulfate was confirmed, 115.03 g of NaSS powder and 0.70 g of 4-ethoxyphenol were added to the reactor, and a cation exchange reaction was performed while stirring the mixture at an internal temperature of 48°C for 60 minutes. Thereafter, the mixture was cooled to 25°C over 5 hours and aged in that state for 2 hours to obtain a white slurry liquid.

Subsequently, the slurry liquid was subjected to centrifugal filtration (600G × 20 minutes, normal temperature) to obtain 82.85 g of plate-shaped wet crystals. The slurry had a good deliquoring properties, and the moisture of the wet crystal determined by an infrared moisture meter was 5.97% by weight.

The wet crystal was dried at 40°C for 30 minutes using a rotary evaporator (pressure: 660 Pa) to obtain 77.82 g of dried crystals (moisture: 0.41% by weight).

From the analysis results of the contents of sodium and nitrogen in the dried crystals (Table 8), the dried crystals were determined to be the target AmSS composition.

The AmSS content of the dried crystals, determined by redox titration, i.e., purity, was 97.9% by weight (yield based on the molar amount of raw material NaSS: 77%).

It is apparent that the alkali metal component, the halogen component, and the polymer component in the AmSS composition are smaller and higher in purity than those in Comparative Examples 1 to 2, Comparative Examples 4 to 5, Comparative Example 7, and Comparative Example 11 (Tables 5 and 7).

In addition, since the median diameter of AmSS is 362 μm, which is much larger than 11 μm (Table 7) shown in Comparative Example 11, the dust generation properties can be expected to be lower than that of Comparative Example 11.

In addition, since the AmSS composition has less moisture and contains 169 ppm of 4-ethoxyphenol, it is apparent that the AmSS composition has excellent storage stability as compared with Comparative Examples 1 to 4 and Comparative Examples 6 to 7 (Table 5). Furthermore, it is apparent that coloring is much less likely to occur as compared with Comparative Examples 8 to 10 (Table 6) since the lithium component and nitrite component are low.

In addition, the charged charge amount per unit mass of the AmSS composition was 0.090 μC/g, which was slightly lower than that of Comparative Example 11 (Table 7) having a small particle size.

<Example 8> Production of AmSS using NaSS and ammonium sulfate (11) TBC

AmSS was produced under the same conditions as in Example 7 except that the polymerization inhibitor was changed to 4-tert-butylcatechol and the addition amount was reduced. As a result, 83.83 g of plate-shaped wet crystals

were obtained. The slurry had a good deliquoring properties, and the moisture of the wet crystal determined by an infrared moisture meter was 7.16% by weight. The wet crystals were dried using a rotary evaporator to obtain 77.9 g of dried crystals (moisture: 0.37% by weight).

From the analysis results of the contents of sodium and nitrogen in the dried crystals (Table 8), the dried crystals were determined to be the target AmSS composition.

The AmSS content of the dried crystals, determined by redox titration, i.e., purity, was 98.0% by weight (yield based on the molar amount of raw material NaSS: 77%).

It is apparent that the alkali metal component, the halogen component, and the polymer component in the AmSS composition are lower than those in Comparative Examples 1 to 6, Comparative Example 7, and Comparative Example 11 (Tables 5 and 7), and are of high purity.

In addition, since the median diameter of AmSS is 345 μm, which is much larger than 11 μm (Table 7) shown in Comparative Example 11, the dust generation properties can be expected to be lower than that of Comparative Example 11.

In addition, since the AmSS composition has less moisture and contains 38 ppm of 4-tert-butylcatechol, it is apparent that the AmSS composition has excellent storage stability as compared with Comparative Examples 8 to 10 (Table 6). Furthermore, since it has a low lithium component and a low nitrite component, and has a low content of 4-tert-butylcatechol, which and is easily colored, it is apparent that coloring is difficult as compared with Comparative Examples 8 to 10 (Table 6).

In addition, the charged charge amount per unit mass of the AmSS composition was 0.091 μC/g, which was slightly lower than that of Comparative Example 11 (Table 7) having a small particle size.

<Example 9> Production of AmSS using NaSS and ammonium sulfate (12) H-TEMPO

AmSS was produced under the same conditions as in Example 8 except that the polymerization inhibitor was changed to 4-hydroxy-2,2,6,6-tetramethylpiperidine 1-oxyl, and as a result, 85.74 g of a plate-shaped wet crystals was obtained. The slurry had a good deliquoring properties, and the moisture of the wet crystal determined by an infrared moisture meter was 7.91% by weight. The wet crystals were dried using a rotary evaporator to obtain 79.28 g of dried crystals (moisture: 0.40% by weight).

From the analysis results of the contents of sodium and nitrogen in the dried crystals (Table 8), the dried crystals were determined to be the target AmSS composition.

The AmSS content of the dried crystals, determined by redox titration, i.e., purity, was 97.8% by weight (yield based on the molar amount of raw material NaSS: 78%).

It is apparent that the alkali metal component, the halogen component, and the polymer component in the AmSS composition are smaller and higher in purity than those in Comparative Examples 1 to 2, Comparative Examples 4 to 5, Comparative Example 7, and Comparative Example 11 (Tables 5 and 7).

In addition, since the median diameter of AmSS is 350 μm, which is much larger than 11 μm (Table 7) shown in Comparative Example 11, the dust generation properties can be expected to be lower than that of Comparative Example 11.

In addition, since the AmSS composition has less moisture and contains 43 ppm of 4-hydroxy-2,2,6,6-tetramethylpiperidine-1-oxyl which has a high polymerization inhibiting ability, it is apparent that the AmSS composition has excellent storage stability as compared with Comparative Examples 1 to 4 and Comparative Examples 6 to 7 (Table 5). Furthermore, since the lithium component and nitrite component are low and the content of colored 4-hydroxy -2,2,6,6-tetramethylpiperidine 1-oxyl is low, it is apparent that coloring is difficult as compared with Comparative Examples 8 to 10 (Table 6).

In addition, the charged charge amount per unit mass of the AmSS composition was 0.091 μC/g, which was slightly lower than that of Comparative Example 11 (Table 7) having a small particle size.

[Table 8]

Table 8

| | | Example 7 | Example 8 | Example 9 |
|---|---|---|---|---|
| [Composition to be charged] | | | | |
| Sodium styrenesulfonate | g | 115.03 | 115.03 | 115.00 |
| (NaSS) | mol | 0.49 | 0.49 | 0.49 |
| Polymerization inhibitor* | | ETPE | TBC | H-TEMPO |

(continued)

| | | Example 7 | Example 8 | Example 9 |
|---|---|---|---|---|
| | g | 0.70 | 0.25 | 0.20 |
| (With respect to NaSS) mol% | | 1.03 | 0.31 | 0.24 |
| Ammonium sulfate | g | 73.01 | 72.86 | 72.82 |
| | mol | 0.55 | 0.55 | 0.55 |
| Ion-exchanged water | g | 255.00 | 255.00 | 255.00 |
| NH4/NaSS molar ratio | Equivalent | 2.22 | 2.22 | 2.22 |
| Total solids content | wt% | 40.67 | 40.59 | 40.57 |
| [Temperature conditions] | | | | |
| Heating/reaction temperature | °C | 48 | 48 | 50 |
| Cooling/filtration temperature | °C | 25 | 25 | 25 |
| [AmSS crystal recovery conditions] | | Centrifugal filtration room temperature x 20 minutes | | |
| [AmSS crystal drying conditions] | | Rotary evaporator 40°C×30 minutes at 660Pa | | |
| [Results] | | | | |
| AmSS composition(Dried product) | g | 77.82 | 77.90 | 79.28 |
| Purity | wt% | 97.9 | 98.0 | 97.8 |
| Yield based on the molar amount of raw material NaSS | % | 77 | 77 | 78 |
| Moisture(Target < 8.0) | wt% | 0.41 | 0.37 | 0.40 |
| Inhibitor content | ppm | 169 | 38 | 43 |
| Na content | wt% | 0.31 | 0.30 | 0.40 |
| Li content | ppm | ≤1 | ≤1 | ≤1 |
| Halogen content | wt% | 0.06 | 0.06 | 0.07 |
| Nitrite content | ppm | 12 | 13 | 13 |
| Polymer component | wt% | 0.00 | 0.00 | 0.00 |
| Total sulfur component | wt% | 16.0 | 15.9 | 15.9 |
| Total nitrogen component | wt% | 7.0 | 6.9 | 7.0 |
| Median diameter | μm | 362 | 345 | 350 |
| [Storage stability] | | | | |
| Polymer component after storage at 60°C for 10 days | wt% | 0.00 | 0.00 | 0.00 |
| Polymer component after storage at 60°C for 60 days | wt% | 0.01 | 0.04 | 0.00 |
| APHA* after 60°C x 60 days | | 69 | 97 | 85 |
| *ETPE:4-ethoxyphenol, TBC:4-tert-butylcatechol<br>H-TEMPO:4-hydroxy-2,2,6,6-tetramethylpiperidine1-oxyl<br>**AmSS composition (as prepared) 10 wt% aqueous solution | | | | |

<Example 10> Production of AmSS using NaSS and ammonium nitrate (1) H-TEMPO

Into a cylindrical 0.5 L glass separable flask equipped with a reflux condenser were charged 89.00 g of ammonium nitrate and 285.00 g of ion-exchanged water, which were dissolved while being heated to an internal temperature of 40°C under stirring. After the dissolution of ammonium nitrate was confirmed, 115.04 g of NaSS powder and 0.25 g of 4-hydroxy -2,2,6,6-tetramethylpiperidine 1-oxyl were added to the reactor, and a cation exchange reaction was performed while stirring the mixture at an internal temperature of 50°C for 30 minutes. Thereafter, the mixture was

cooled to 20°C over 5 hours and aged in that state for 2 hours to obtain a white slurry liquid.

Subsequently, the slurry liquid was subjected to centrifugal filtration (600G × 20 minutes, normal temperature) to obtain 81.75 g of plate-shaped wet crystals. The slurry had a good deliquoring properties, and the moisture of the wet crystal determined by an infrared moisture meter was 6.55% by weight.

The wet crystal was dried at 40°C for 30 minutes using a rotary evaporator (pressure: 660 Pa) to obtain 76.60 g of dried crystals (moisture: 0.85% by weight).

From the analysis results of the contents of sodium and nitrogen in the dried crystals (Table 9), the dried crystals were determined to be the target AmSS composition.

The AmSS content of the dried crystals, determined by redox titration, i.e., purity, was 96.5% by weight (yield based on the molar amount of raw material NaSS: 75%).

It is apparent that the alkali metal component, the halogen component, and the polymer component in the AmSS composition are smaller and higher in purity than those in Comparative Examples 1 to 2, Comparative Examples 4 to 5, Comparative Example 7, and Comparative Example 11 (Tables 5 and 7).

In addition, since the median diameter of AmSS is 374 μm, which is much larger than 11 μm (Table 7) shown in Comparative Example 11, the dust generation properties can be expected to be lower than that of Comparative Example 11.

In addition, since the AmSS composition has less moisture and contains 49 ppm of 4-hydroxy-2,2,6,6-tetramethylpiperidine-1-oxyl which has a high polymerization inhibiting ability, it is apparent that the AmSS composition has excellent storage stability as compared with Comparative Examples 1 to 4 and Comparative Examples 6 to 7 (Table 5). Furthermore, it is apparent that coloring is less likely to occur as compared with Comparative Examples 8 to 10 (Table 6) since the lithium component and nitrite component are low.

In addition, the charged charge amount per unit mass of the AmSS composition was 0.088 μC/g, which was slightly lower than that of Comparative Example 11 (Table 7) having a small particle size.

<Example 11> Production of AmSS using NaSS and ammonium nitrate (2) MEPE

Into a cylindrical 0.5 L glass separable flask equipped with a reflux condenser were charged 89.00 g of ammonium nitrate and 239.70 g of ion-exchanged water, which were dissolved while being heated to an internal temperature of 40°C under stirring. After the dissolution of ammonium nitrate was confirmed, 115.04 g of NaSS powder and 1.20 g of 4-methoxyphenol were added to the reactor, and a cation exchange reaction was performed while stirring the mixture at an internal temperature of 55°C for 30 minutes. Thereafter, the mixture was cooled to 20°C over 5 hours and aged in that state for 2 hours to obtain a white slurry liquid.

Subsequently, the slurry liquid was subjected to centrifugal filtration (600G × 20 minutes, normal temperature) to obtain 91.32 g of plate-shaped wet crystals. The slurry had a good deliquoring properties, and the moisture of the wet crystal determined by an infrared moisture meter was 5.83% by weight.

The wet crystal was dried at 40°C for 30 minutes using a rotary evaporator (pressure: 660 Pa) to obtain 86.30 g of dried crystals (moisture: 0.88% by weight).

From the analysis results of the contents of sodium and nitrogen in the dried crystals (Table 9), the dried crystals were determined to be the target AmSS composition.

The AmSS content of the dried crystals, determined by redox titration, i.e., purity, was 96.7% by weight (yield based on the molar amount of raw material NaSS: 84%).

It is apparent that the alkali metal component, the halogen component, and the polymer component in the AmSS composition are smaller and higher in purity than those in Comparative Examples 1 to 2, Comparative Examples 4 to 5, Comparative Example 7, and Comparative Example 11 (Tables 5 and 7) described later.

In addition, since the median diameter of AmSS is 341 μm, which is much larger than 11 μm (Table 7) shown in Comparative Example 11, the dust generation properties can be expected to be lower than that of Comparative Example 11.

In addition, since the AmSS composition has less moisture and contains 223 ppm of 4-methoxyphenol, it is apparent that the AmSS composition has excellent storage stability as compared with Comparative Examples 1 to 4 and Comparative Examples 6 to 7 (Table 5). Furthermore, it is apparent that coloring is much less likely to occur as compared with Comparative Examples 8 to 10 (Table 6) since the lithium component and nitrite component are low.

In addition, the charged charge amount per unit mass of the AmSS composition was 0.088 μC/g, which was slightly lower than that of Comparative Example 11 (Table 7) having a small particle size.

<Example 12> Production of AmSS using NaSS and ammonium nitrate (3) MEPE

A cylindrical 0.5 L glass separable flask equipped with a reflux condenser was charged with 103.00 g of NaSS powder, 0.44 g of 4-methoxyphenol, and 142.00 g of ion-exchanged water, and the mixture was stirred and formed into a

slurry while being heated to an internal temperature of 40°C. An aqueous ammonium nitrate solution (obtained by dissolving 105.00 g of ammonium nitrate in 150.00 g of ion-exchanged water) heated to 40°C was added to the vessel, and the mixture was subjected to a cation exchange reaction while being stirred at an internal temperature of 51°C for 30 minutes. Thereafter, the mixture was cooled to 20°C over 5 hours and aged in that state for 2 hours to obtain a white slurry liquid.

Subsequently, the slurry liquid was subjected to centrifugal filtration (600G × 20 minutes, normal temperature) to obtain 82.11 g of plate-shaped wet crystals. The slurry had a good deliquoring properties, and the moisture of the wet crystal determined by an infrared moisture meter was 7.75% by weight.

The wet crystal was dried at 40°C for 30 minutes using a rotary evaporator (pressure: 660 Pa) to obtain 76.41 g of dried crystals (moisture: 1.62% by weight).

From the analysis results of the contents of sodium and nitrogen in the dried crystals (Table 9), the dried crystals were determined to be the target AmSS composition.

The AmSS content of the dried crystals, determined by redox titration, i.e., purity, was 94.5% by weight (yield based on the molar amount of raw material NaSS: 81%).

It is apparent that the alkali metal component, the halogen component, and the polymer component in the AmSS composition are smaller and higher in purity than those in Comparative Examples 1 to 2, Comparative Examples 4 to 5, Comparative Example 7, and Comparative Example 11 (Tables 5 and 7) described later.

In addition, since the median diameter of AmSS is 390 μm, which is much larger than 11 μm (Table 7) shown in Comparative Example 11, the dust generation properties can be expected to be lower than those of Comparative Example 11.

In addition, since the AmSS composition has less moisture and contains 280 ppm of 4-methoxyphenol, it is apparent that the AmSS composition has excellent storage stability as compared with Comparative Examples 1 to 4 and Comparative Examples 6 to 7 (Table 5). Furthermore, it is apparent that coloring is much less likely to occur as compared with Comparative Examples 8 to 10 (Table 6) since the lithium component and nitrite component are low.

In addition, the charged charge amount per unit mass of the AmSS composition was 0.084 μC/g, which was slightly lower than that of Comparative Example 11 (Table 7) having a small particle size.

<Example 13> Production of AmSS using NaSS and ammonium nitrate (4) MEPE

A cylindrical 0.5 L glass separable flask equipped with a reflux condenser was charged with NaSS powder 115.04.00g, 0.49 g of 4-methoxyphenol, and 115.00 g of ion-exchanged water, and the mixture was stirred and formed into a slurry while being heated to an internal temperature of 40°C. An aqueous ammonium nitrate solution (obtained by dissolving 89.00 g of ammonium nitrate in 90 g of ion-exchanged water) heated to 40°C was added to the vessel, and the mixture was subjected to a cation exchange reaction while being stirred at an internal temperature of 60°C for 60 minutes. Thereafter, the mixture was cooled to 20°C over 5 hours and aged in that state for 2 hours to obtain a white slurry liquid.

Subsequently, the slurry liquid was subjected to centrifugal filtration (600G × 20 minutes, normal temperature) to obtain 99.74 g of plate-shaped wet crystals. The slurry had a good deliquoring properties, and the moisture of the wet crystal determined by an infrared moisture meter was 7.45% by weight.

The wet crystal was dried at 40°C for 30 minutes using a rotary evaporator (pressure: 660 Pa) to obtain 93.48 g of dried crystals (moisture: 1.31% by weight).

From the analysis results of the contents of sodium and nitrogen in the dried crystals (Table 9), the dried crystals were determined to be the target AmSS composition.

The AmSS content of the dried crystals, determined by redox titration, i.e., purity, was 95.3% by weight (yield based on the molar amount of raw material NaSS: 90%).

It is apparent that the alkali metal component, the halogen component, and the polymer component in the AmSS composition are smaller and higher in purity than those in Comparative Examples 1 to 2, Comparative Examples 4 to 5, Comparative Examples 6 to 7, and Comparative Example 11 (Tables 5 and 7) described later.

In addition, since the median diameter of AmSS is 386 μm, which is much larger than 11 μm (Table 7) shown in Comparative Example 11, the dust generation properties can be expected to be lower than those of Comparative Example 11.

In addition, since the AmSS composition has less moisture and contains 412 ppm of 4-methoxyphenol, it is apparent that the AmSS composition has excellent storage stability as compared with Comparative Examples 1 to 4 and Comparative Examples 6 to 7 (Table 5). Furthermore, it is apparent that coloring is much less likely to occur as compared with Comparative Examples 8 to 10 (Table 6) since the lithium component and nitrite component are low.

In addition, the charged charge amount per unit mass of the AmSS composition was 0.079 μC/g, which was slightly lower than that of Comparative Example 11 (Table 7) having a small particle size.

<Comparative Example 12> Production of AmSS using NaSS and ammonium nitrate (5) MEPE

A cylindrical 0.5 L glass separable flask equipped with a reflux condenser was charged with 115.01 g of NaSS powder, 1.20 g of 4-methoxyphenol, 89.00 g of ammonium nitrate, and 165.00 g of ion-exchanged water; the mixture was subjected to a cation exchange reaction while being stirred at an internal temperature of 60°C for 60 minutes. Thereafter, the mixture was cooled to 20°C over 5 hours and aged in that state for 2 hours to obtain a white slurry liquid.

Subsequently, the slurry liquid was subjected to centrifugal filtration (600G × 20 minutes, normal temperature) to obtain 110.00 g of plate-shaped wet crystals, but the slurry had a poor deliquoring properties, and the moisture of the wet crystals as determined by an infrared moisture meter was 15.50% by weight.

The wet crystal was dried at 40°C for 30 minutes using a rotary evaporator (pressure: 660 Pa) to obtain 92.67 g of dried crystals (moisture: 1.98% by weight).

The AmSS content of the dried crystals determined by redox titration, that is, the purity was 92.7% by weight (the yield based on the mole of the raw material NaSS was 95%), but the sodium component determined by ICP was 0.65% by weight, which was significantly higher than that in Example 13. This is because the total solids content during the reaction was too high.

In addition, the charged charge amount per unit mass of the AmSS composition was 0.078 μC/g, which was slightly lower than that of Comparative Example 11 (Table 7) having a small particle size.

[Table 9]

Table 9

| | | Example 10 | Example 11 | Example 12 | Example 13 | Comparative Example 12 |
|---|---|---|---|---|---|---|
| [Composition to be charged] | | | | | | |
| Sodium styrenesulfonate | g | 115.04 | 115.04 | 103.00 | 115.04 | 115.01 |
| (NaSS) | mol | 0.49 | 0.49 | 0.44 | 0.49 | 0.49 |
| Polymerization inhibitor* | | H-TEMPO | MEPE | MEPE | MEPE | MEPE |
| | g | 0.25 | 1.20 | 1.10 | 1.20 | 1.20 |
| (With respect to NaSS) | mol% | 0.29 | 1.96 | 2.01 | 1.96 | 1 96 |
| Ammonium nitrate | g | 89.00 | 89.00 | 105.00 | 89.00 | 89.00 |
| | mol | 1.10 | 1.10 | 1.30 | 1.10 | 1.10 |
| Ion-exchanged water | g | 285.00 | 239.70 | 292.00 | 205.00 | 165.00 |
| NH4/NaSS molar ratio | Equivalent | 2.24 | 2.24 | 2.95 | 2.24 | 2.24 |
| Total solids content | wt% | 40.06 | 44.27 | 40.25 | 48.01 | 53.19 |
| [Temperature conditions] | | | | | | |
| Heating/reaction temperature | °C | 50 | 55 | 51 | 60 | 60 |
| Coolina/filtration temperature | °C | 20 | 20 | 20 | 20 | 20 |
| [AmSS crystal recovery conditions] | | Centrifugal filtration room temperature x 20 minutes | | | | |
| [AmSS crystal drying conditions] | | Rotary evaporator 40°C × 30 minutes at 660Pa | | | | |
| [Results] | | | | | | |
| AmSS composition(Dried product) | g | 76.60 | 86.30 | 76.41 | 93.48 | 101.20 |
| Purity | wt% | 96.5 | 96.7 | 94.5 | 95.3 | 92.7 |
| Yield based on the molar amount of raw material NaSS | % | 75 | 84 | 81 | 90 | 95 |

(continued)

| | | Example 10 | Example 11 | Example 12 | Example 13 | Comparative Example 12 |
|---|---|---|---|---|---|---|
| Moisture(Target < 8.0) | wt% | 0.85 | 0.88 | 1.62 | 1.31 | 1.98 |
| Inhibitor content | ppm | 49 | 223 | 280 | 412 | 1957 |
| Na content | wt% | 0.20 | 0.20 | 0.32 | 0.33 | 065 |
| Li content | ppm | ≤1 | ≤1 | ≤1 | ≤1 | |
| Halogen content | wt% | 0.04 | 0.04 | 0.06 | 0.06 | 0.10 |
| Nitrite content | ppm | 10 | 9 | 12 | 12 | 19 |
| Nitrate ion content | wt% | 1.40 | 1.40 | 2.50 | 2.10 | 4.19 |
| Polymer component | wt% | 0.00 | 0.00 | 0.00 | 0.00 | 0.01 |
| Total sulfur component | wt% | 15.5 | 15.5 | 15.1 | 15.3 | 14.7 |
| Total nitrogen component | wt% | 7.3 | 7.3 | 7.6 | 7.5 | 8.0 |
| Median diameter | μm | 374 | 341 | 390 | 386 | 305 |
| [Storage stability] | | | | | | |
| Polymer component after storage at 60°C for 10 days | wt% | 0.00 | 0.00 | 0.00 | 0.00 | 0.00 |
| Polymer component after storage at 60°C for 60 days | wt% | 0.02 | 0.01 | 0.02 | 0.02 | 0.04 |
| APHA* after 60°C x 60 days | | 79 | 68 | 74 | 73 | 88 |
| * H-TEMPO:4-hydroxy-2,2,6,6-tetramethylpiperidinel-oxyi<br>MEPE:4-Methoxyphenol<br>**AmSS composition (as prepared) 10 wt% aqueous solution | | | | | | |

<Reference Examples 1 to 4> Filterability of AmSS solution

In a 100 ml stoppered Erlenmeyer flask, the AmSS composition obtained in each of Examples 1, 3, and 10 and Comparative Example 11 and N-methylpyrrolidone were collected in the composition shown in Table 10, and the AmSS composition was dissolved using a magnetic stirrer while immersing the AmSS composition in a hot water bath at 35°C. After visually confirming the dissolution, the solution was immediately filtered with a Buchner funnel (Quantitative filter paper No. 5A manufactured by Advantec Toyo Roshi Kaisha, Ltd., diameter: 70 mm, operation pressure: 3.0 kPa), and the filtration time at that time was measured.

As shown in Table 10, it is apparent that the lower the sodium component, the shorter the filtration time. That is, the lower the metal component such as sodium, the more preferable it is for electronic material applications, but it is also advantageous in terms of filterability.

[Table 10]

Table 10

| | | Reference Example 1 | Reference Example 2 | Reference Example 3 | Reference Example 4 |
|---|---|---|---|---|---|
| [NMP solution] | | | | | |
| AmSS composition | | Example 1 | Example 10 | Example 3 | Comparative Example 11 |
| Purity | wt% | 90.6 | 96.5 | 89.9 | 98.0 |
| Moisture | wt% | 8.00 | 0.85 | 8.41 | 0.50 |
| Na content | wt% | 0.27 | 0.20 | 0.45 | 5.30 |
| AmSS composition | g | 15.20 | 15.01 | 15.30 | 14.02 |

(continued)

| | | Reference Example 1 | Reference Example 2 | Reference Example 3 | Reference Example 4 |
|---|---|---|---|---|---|
| NMP | g | 49.99 | 50.00 | 49.70 | 51.03 |
| Pure AmSS concentration | wt% | 21.12 | 22.28 | 21.16 | 21.12 |
| [Filtration time] | Second | 2 | 2 | 10 | 68 |

## Claims

1. An ammonium styrenesulfonate composition having characteristics (1) to (6) shown below:

    (1) the ammonium styrenesulfonate composition has an ammonium styrenesulfonate content of 88.0% by weight or more;
    (2) the ammonium styrenesulfonate composition has a water content of 10.00% by weight or less;
    (3) the ammonium styrenesulfonate composition has an alkali metal content of 0.50% by weight or less;
    (4) the ammonium styrenesulfonate composition has a halogen content of 1.00% by weight or less;
    (5) the ammonium styrenesulfonate composition has a polymer content of 0.20% by weight or less; and
    (6) the ammonium styrenesulfonate composition has a polymerization inhibitor content of 2000 ppm or less.

2. The ammonium styrenesulfonate composition according to claim 1, wherein

    (1) the ammonium styrenesulfonate composition has an ammonium styrenesulfonate content of 88.0% by weight or more;
    (2) the ammonium styrenesulfonate composition has a water content of 0.10% by weight to 10.00% by weight;
    (3) the ammonium styrenesulfonate composition has an alkali metal content of 0.50% by weight or less;
    (4) the ammonium styrenesulfonate composition has a halogen content of 1.00% by weight or less;
    (5) the ammonium styrenesulfonate composition has a polymer content of 0.20% by weight or less; and
    (6) the ammonium styrenesulfonate composition has a polymerization inhibitor content of 20 ppm to 2000 ppm.

3. The ammonium styrenesulfonate composition according to claim 1, wherein

    (1) the ammonium styrenesulfonate composition has an ammonium styrenesulfonate content of 94.00% by weight or more;
    (2) the ammonium styrenesulfonate composition has a water content of 0.10% by weight to 6.00% by weight;
    (3) the ammonium styrenesulfonate composition has an alkali metal content of 0.50% by weight or less;
    (4) the ammonium styrenesulfonate composition has a halogen content of 0.10% by weight or less;
    (5) the ammonium styrenesulfonate composition has a polymer content of 0.20% by weight or less; and
    (6) the ammonium styrenesulfonate composition has a polymerization inhibitor content of 20 ppm to 1000 ppm.

4. The ammonium styrenesulfonate composition according to any one of claims 1 to 3, wherein the polymerization inhibitor is at least one selected from the group consisting of 2-methoxyphenol, 3-methoxyphenol, 4-methoxyphenol, 4-ethoxyphenol, 4-cyanophenol, 4-butoxyphenol, 3-ethoxyphenol, 2,5-dimethoxyphenol, 2,6-dimethoxyphenol, 2,6-di-tert-butylphenol, 2,4-di-tert-butylphenol, 2,6-di-tert-butyl-4-methylphenol, 4-tert-butylcatechol, hydroquinone, methylhydroquinone, 2-methoxyhydroquinone, tert-butylhydroquinone, ammonium N-nitrosophenylhydroxylamine, 4-hydroxy-2,2,6,6-tetramethylpiperidine 1-oxyl, 4-(2-hydroxypropoxy-3-(2-hydroxyethoxy))-2,2,6,6-tetramethylpiperidine-1-ol, 4-(3-hydroxypropoxy-2-(2-hydroxyethoxy))-2,2,6,6-tetramethylpiperidine-1-ol, and salicylic acid hydrazide.

5. The ammonium styrenesulfonate composition according to any one of claims 1 to 3, wherein the polymerization inhibitor is at least one phenolic compound selected from the group consisting of 2-methoxyphenol, 3-methoxyphenol, 4-methoxyphenol, 4-ethoxyphenol, 4-cyanophenol, 4-butoxyphenol, 3-ethoxyphenol, 2,5-dimethoxyphenol, and 2,6-dimethoxyphenol.

6. The ammonium styrenesulfonate composition according to any one of claims 1 to 3, wherein a charge amount per unit

mass is 0.020 μC/g to 0.200 μC/g.

**7.** The ammonium styrenesulfonate composition according to any one of claims 1 to 3, wherein a crystal in the composition has a median diameter of 30 μm to 700 μm.

**8.** The ammonium styrenesulfonate composition according to any one of claims 1 to 3, wherein a crystal in the composition has a median diameter of 30 μm to 500 μm.

**9.** The ammonium styrenesulfonate composition according to any one of claims 1 to 3, wherein the polymer content in the composition is 0.20% by weight or less when the composition is stored at 60°C for 60 days in a sealed state.

**10.** The ammonium styrenesulfonate composition according to any of claims 1 to 3, wherein the composition has diffraction peaks at least at diffraction angles $2\theta = 8.1 \pm 0.2°$, $15.2 \pm 0.2°$, $18.4 \pm 0.2°$, $20.6 \pm 0.2°$, $24.2 \pm 0.2°$, $32.5 \pm 0.2°$, and $43.0 \pm 0.2°$ in a powder X-ray diffraction pattern measured by irradiation with copper Kα X-ray.

**11.** The ammonium styrenesulfonate composition according to any of claims 1 to 3, wherein the composition has diffraction peaks at least at diffraction angles $2\theta = 8.1 \pm 0.2°$, $15.2 \pm 0.2°$, $15.4 \pm 0.2°$, $18.4 \pm 0.2°$, $20.1 \pm 0.2°$, $20.6 \pm 0.2°$, $20.8 \pm 0.2°$, $24.2 \pm 0.2°$, $25.8 \pm 0.2°$, $27.5 \pm 0.2°$, $30.5 \pm 0.2°$, $32.5 \pm 0.2°$, $37.5 \pm 0.2°$, $43.0 \pm 0.2°$ and $49.6 \pm 0.2°$ in a powder X-ray diffraction pattern measured by irradiation with copper Kα X-ray.

**12.** The method for producing an ammonium styrenesulfonate composition according to any one of claims 1 to 3, the method comprising bringing sodium or potassium styrenesulfonate into contact with an inorganic ammonium salt in water in the presence of a polymerization inhibitor in an amount of 7 mol% or less with respect to the amount of sodium or potassium styrenesulfonate to cause a cation exchange reaction, and then cooling to precipitate crystals of ammonium styrenesulfonate, and separating the crystals by filtration,
wherein

the charging ratio of ammonium cations to alkali metal styrenesulfonate is 1.50 equivalents to 3.00 equivalents, and
the total solids content in the reaction system is from 25.00% by weight to 50.00% by weight,
a temperature at which sodium or potassium styrenesulfonate is brought into contact with an inorganic ammonium salt is 30°C to 80°C, and
a temperature at which precipitated crystals are separated by filtration is 5°C to 30°C.

**13.** The method for producing the ammonium styrenesulfonate composition according to claim 12,

the method comprising bringing sodium or potassium styrenesulfonate into contact with an inorganic ammonium salt in water in the presence of a polymerization inhibitor in an amount of 7 mol% or less with respect to the amount of sodium or potassium styrenesulfonate to cause a cation exchange reaction, and then cooling to precipitate crystals of ammonium styrenesulfonate, and separating the crystals by filtration,
wherein
the charging ratio of ammonium cations to alkali metal styrenesulfonate is 1.50 equivalents to 3.00 equivalents, and
the total solids content in the reaction system is from 25.00% by weight to 45.00% by weight,
a temperature at which sodium or potassium styrenesulfonate is brought into contact with an inorganic ammonium salt is 30°C to 80°C, and
a temperature at which precipitated crystals are separated by filtration is 5°C to 30°C.

**14.** The method for producing the ammonium styrenesulfonate composition according to claim 12,

the method comprising bringing sodium or potassium styrenesulfonate into contact with an inorganic ammonium salt in water in the presence of a polymerization inhibitor in an amount of 5 mol% or less with respect to the amount of sodium or potassium styrenesulfonate to cause a cation exchange reaction, and then cooling to precipitate crystals of ammonium styrenesulfonate, and separating the crystals by filtration,
wherein
the charging ratio of ammonium cations to alkali metal styrenesulfonate is 2.00 equivalents to 2.50 equivalents, and
the total solids content in the reaction system is from 35.00% by weight to 45.00% by weight,

a temperature at which sodium or potassium styrenesulfonate is brought into contact with an inorganic ammonium salt is 40°C to 60°C, and
a temperature at which precipitated crystals are separated by filtration is 15°C to 25°C.

**15.** The method for producing an ammonium styrenesulfonate composition according to claim 12, wherein the inorganic ammonium salt is at least one compound selected from the group consisting of ammonium chloride, ammonium sulfate, ammonium nitrate, and ammonium acetate.

Fig. 1

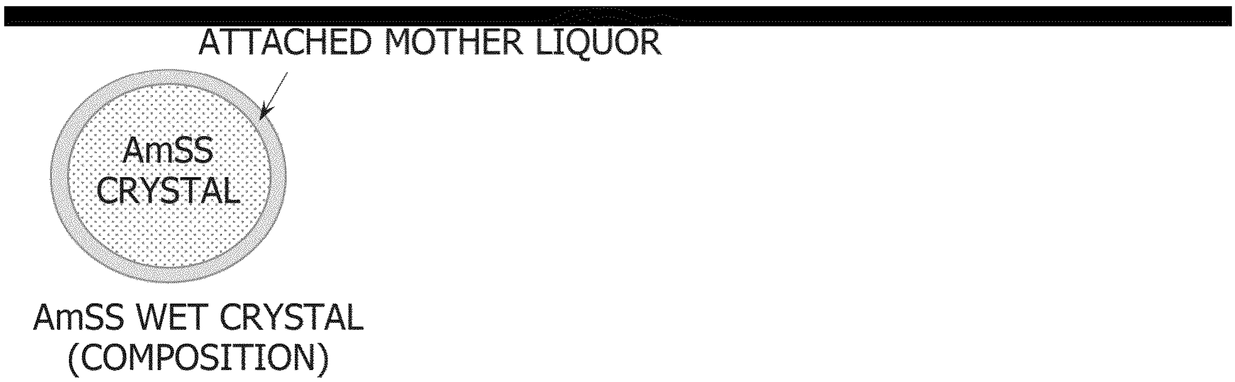
ATTACHED MOTHER LIQUOR
AmSS
CRYSTAL
AmSS WET CRYSTAL
(COMPOSITION)

Fig. 2

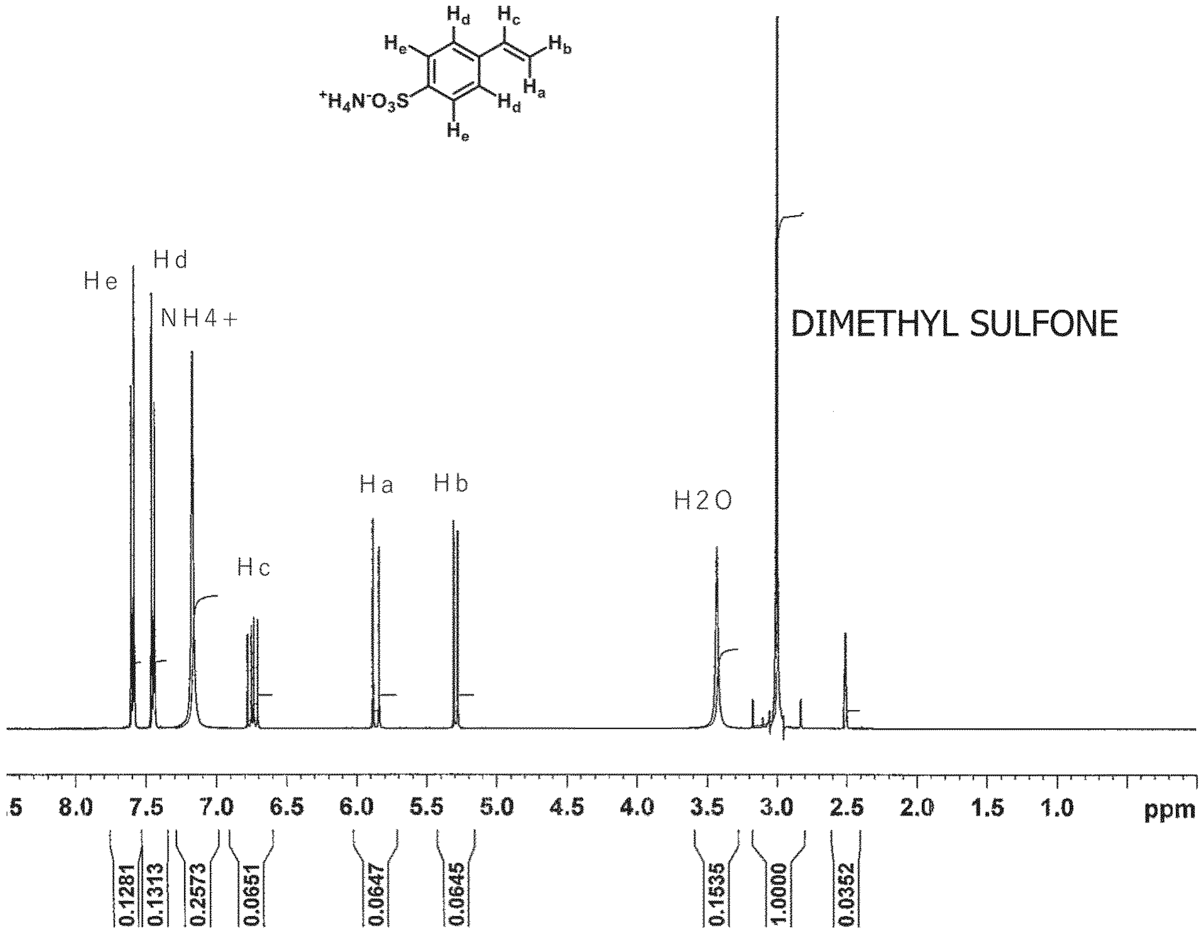
He
Hd
NH4+
Hc
Ha
Hb
H2O
DIMETHYL SULFONE
.5 8.0 7.5 7.0 6.5 6.0 5.5 5.0 4.5 4.0 3.5 3.0 2.5 2.0 1.5 1.0 ppm
0.1281
0.1313
0.2573
0.0651
0.0647
0.0645
0.1535
1.0000
0.0352

Fig. 3

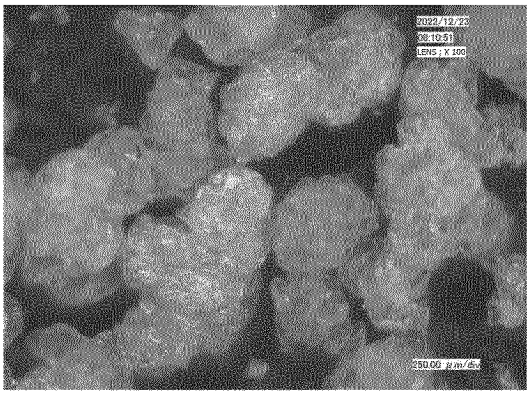
2022/12/23
08:10:51
LENS ; X 100
250.00 μm/div

Fig. 4

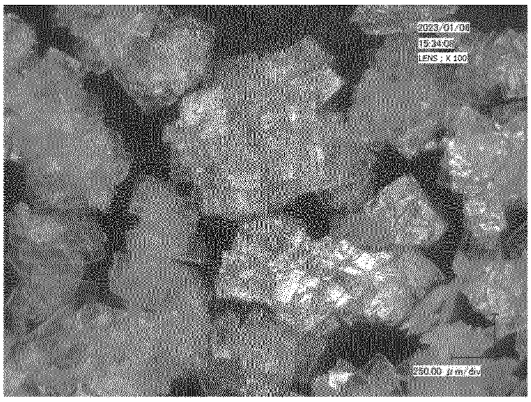
2023/01/06
15:34:08
LENS ; X 100
250.00 μm/div

Fig. 5

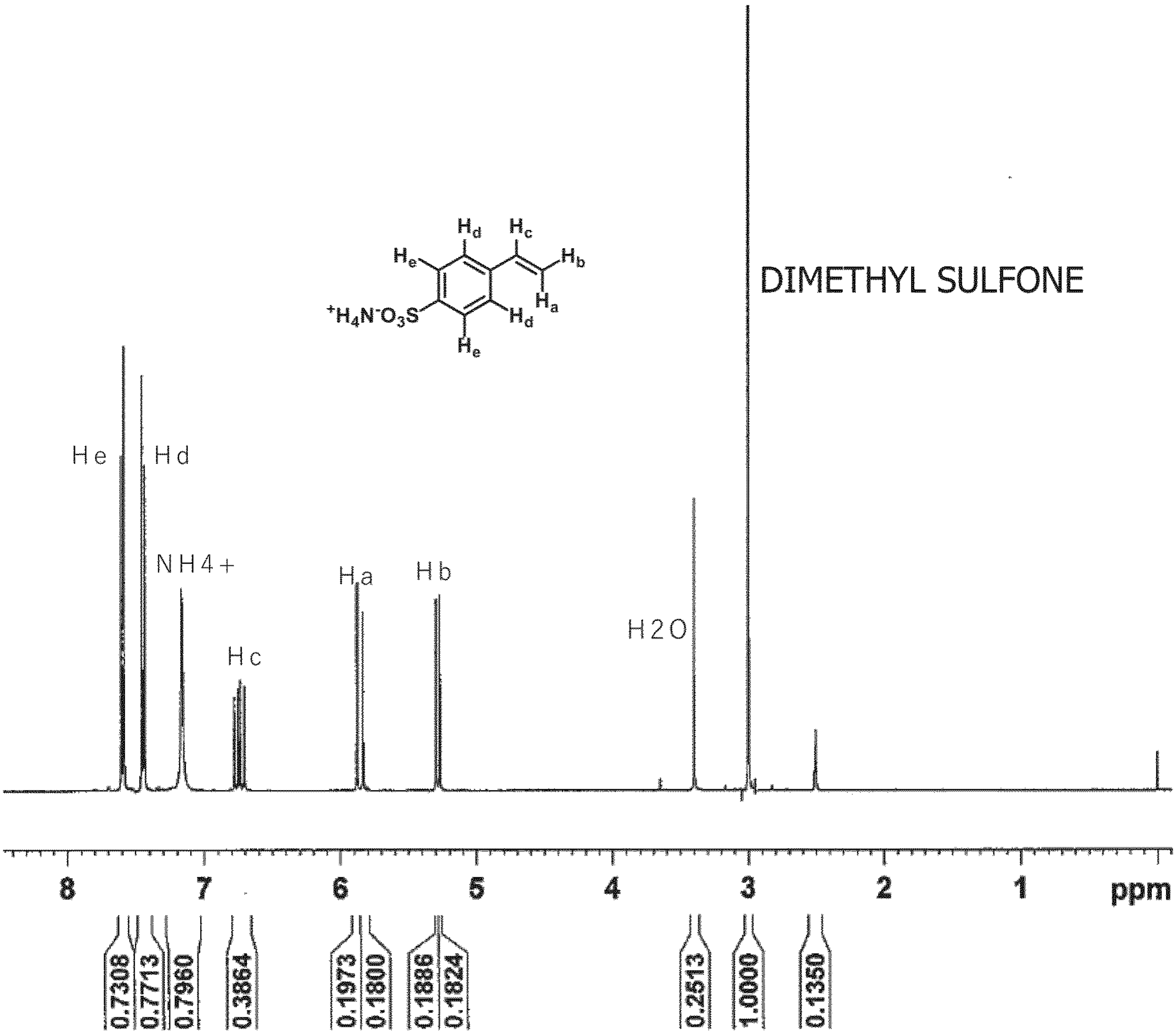
DIMETHYL SULFONE
He
Hd
NH4+
Hc
Ha
Hb
H2O
8
7
6
5
4
3
2
1
ppm
0.7308
0.7713
0.7960
0.3864
0.1973
0.1800
0.1886
0.1824
0.2513
1.0000
0.1350

Fig. 6

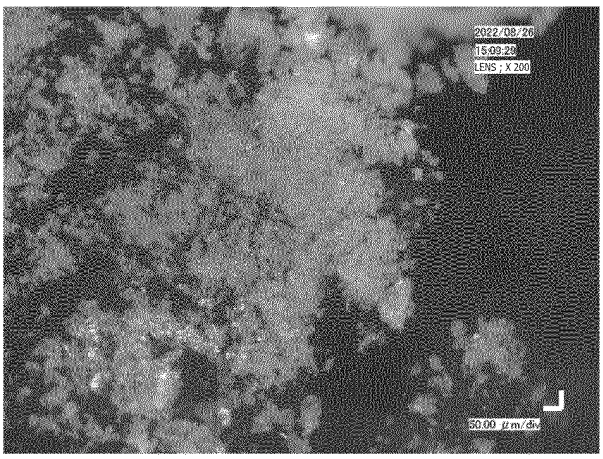
2022/08/26
15:09:28
LENS ; X 200
50.00 μm/div

Fig. 7

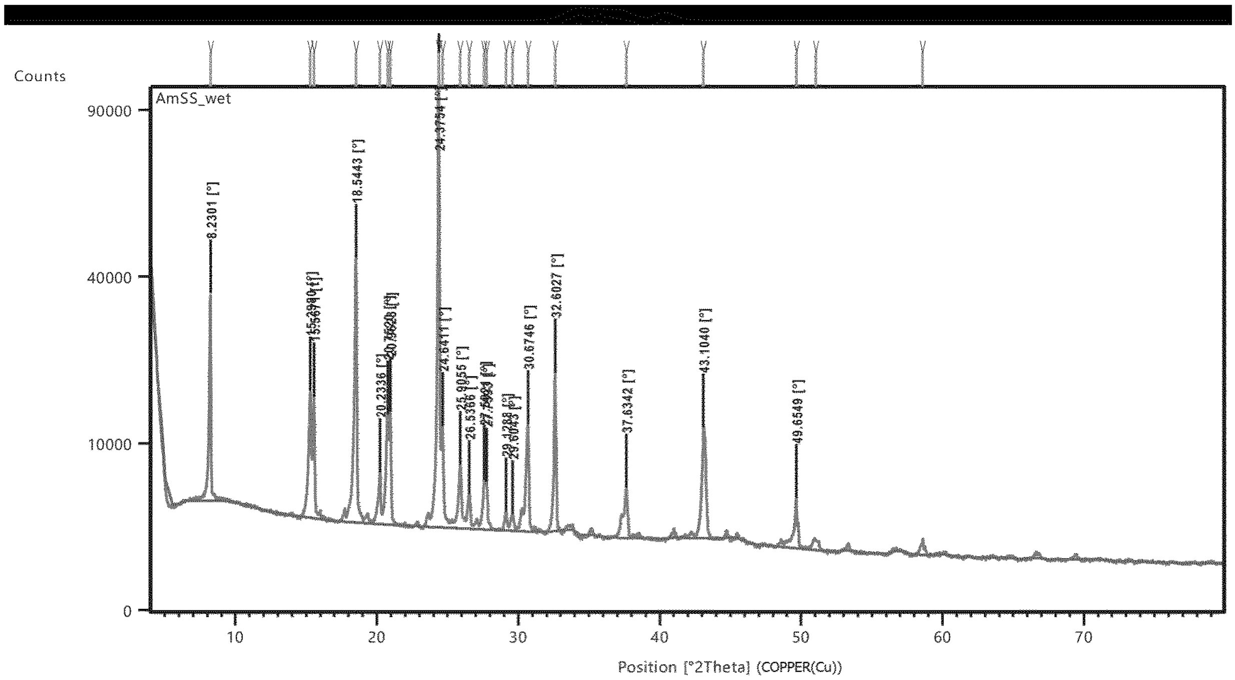
Counts
AmSS_wet
90000
40000
10000
0
8.2301 [°]
15.2980 [°]
15.5671 [°]
18.5443 [°]
20.2336 [°]
20.7520 [°]
20.9623 [°]
24.3754 [°]
24.6411 [°]
25.9055 [°]
26.5366 [°]
27.5921 [°]
27.7593 [°]
29.1288 [°]
29.6043 [°]
30.6746 [°]
32.6027 [°]
37.6342 [°]
43.1040 [°]
49.6549 [°]
10
20
30
40
50
60
70
Position [°2Theta] (COPPER(Cu))

Fig. 8

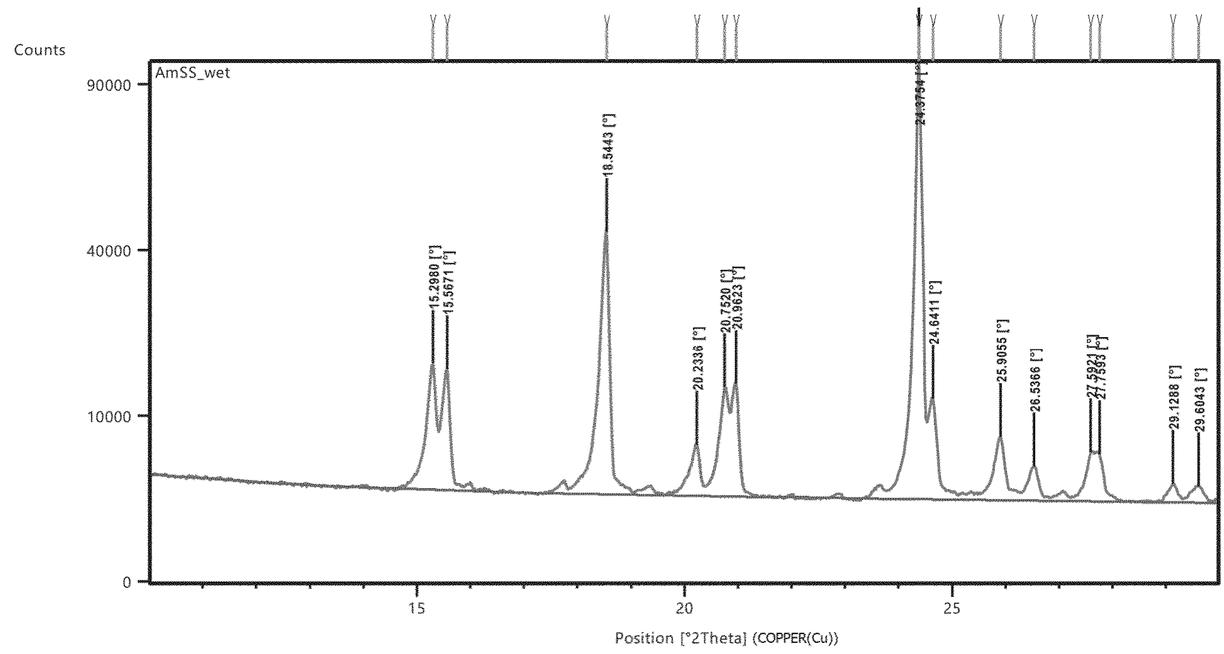
Counts
AmSS_wet
90000
40000
10000
0
15.2980 [°]
15.5671 [°]
18.5443 [°]
20.2336 [°]
20.7520 [°]
20.9623 [°]
24.3754 [°]
24.6411 [°]
25.9055 [°]
26.5366 [°]
27.5921 [°]
27.7593 [°]
29.1288 [°]
29.6043 [°]
15
20
25
Position [°2Theta] (COPPER(Cu))

Fig. 9

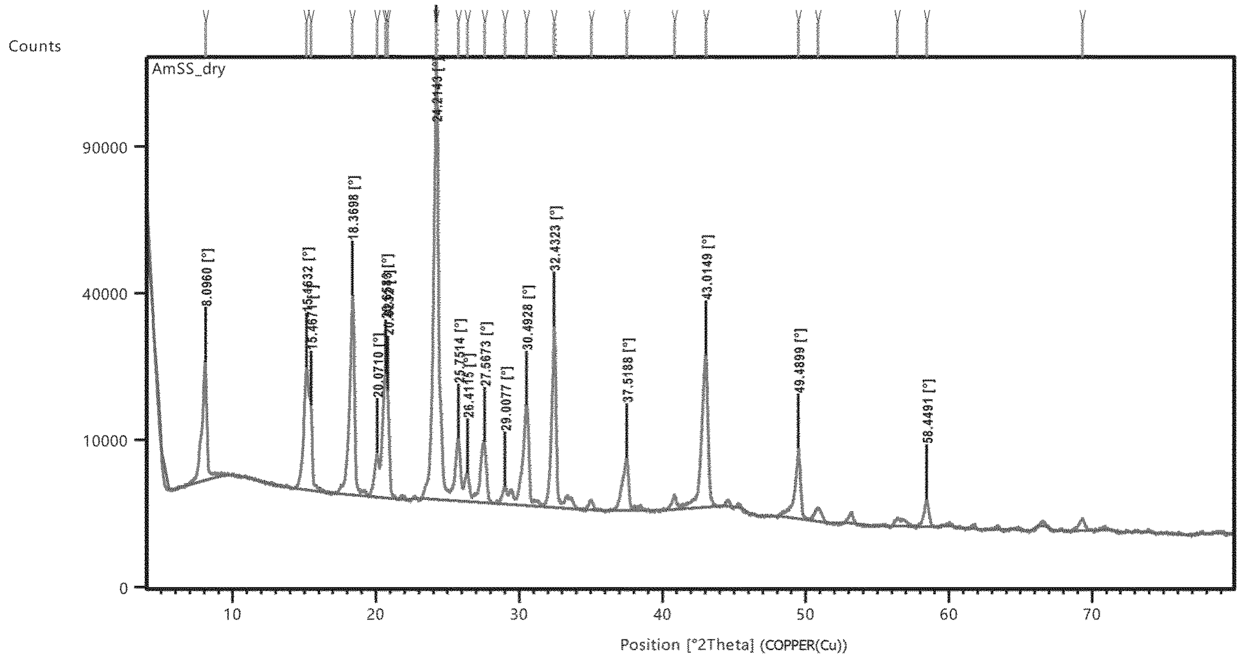
Counts
AmSS_dry
90000
40000
10000
0
10
20
30
40
50
60
70
Position [°2Theta] (COPPER(Cu))
8.0960 [°]
15.1632 [°]
15.4671 [°]
18.3698 [°]
20.0710 [°]
24.2143 [°]
25.7514 [°]
26.4115 [°]
27.5673 [°]
29.0077 [°]
30.4928 [°]
32.4323 [°]
37.5188 [°]
43.0149 [°]
49.4899 [°]
58.4491 [°]

Fig. 10

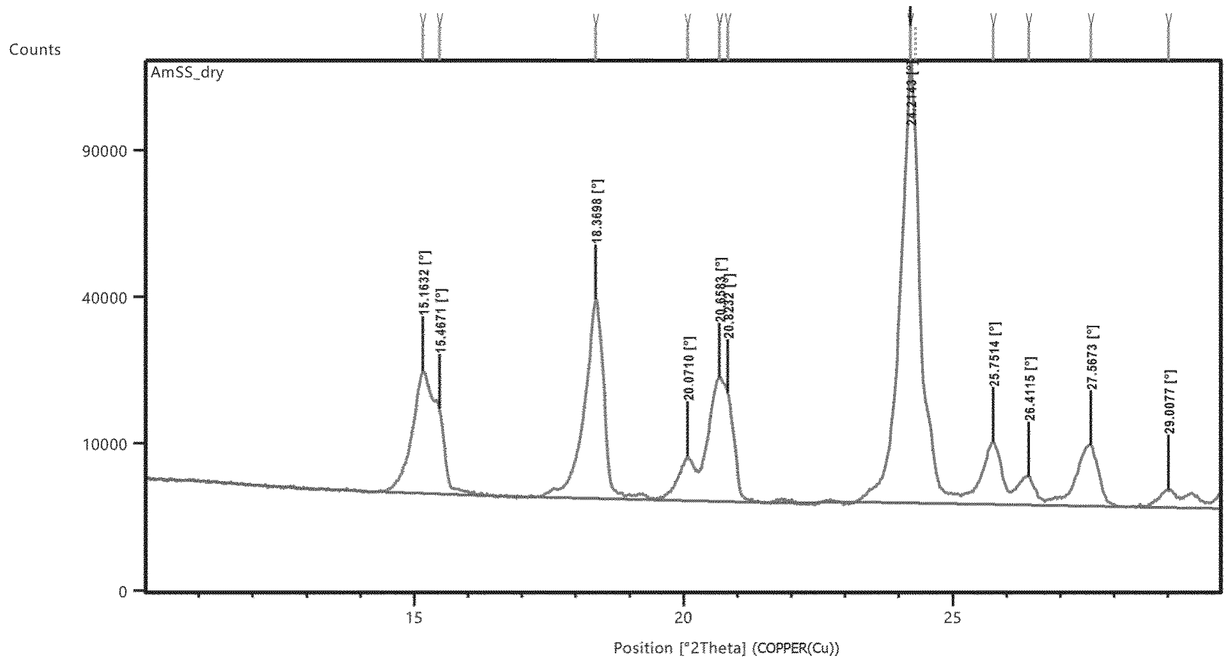
Counts
AmSS_dry
90000
40000
10000
0
15
20
25
Position [°2Theta] (COPPER(Cu))
15.1632 [°]
15.4671 [°]
18.3698 [°]
20.0710 [°]
20.6583 [°]
20.8232 [°]
24.2143 [°]
25.7514 [°]
26.4115 [°]
27.5673 [°]
29.0077 [°]

Fig. 11

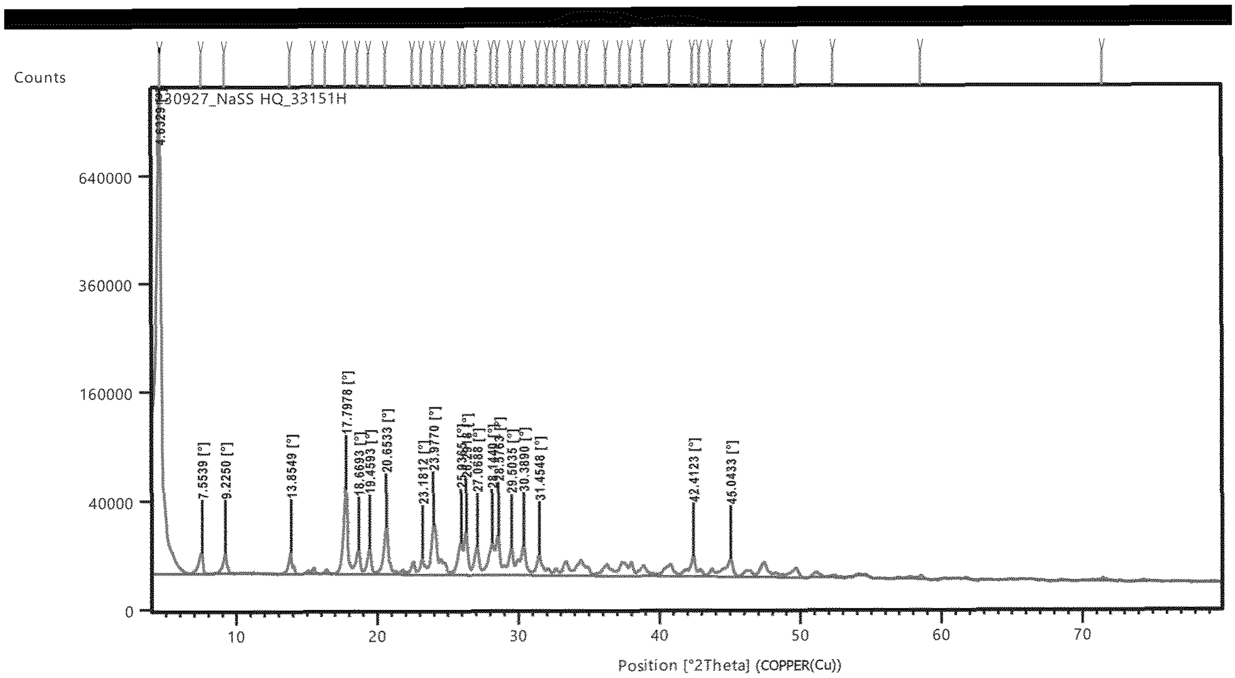
Counts
230927_NaSS HQ_33151H
640000
360000
160000
40000
0
4.6329 [°]
7.5539 [°]
9.2250 [°]
13.8549 [°]
17.7978 [°]
18.6693 [°]
19.4593 [°]
20.6533 [°]
23.1812 [°]
23.9770 [°]
25.9365 [°]
26.2918 [°]
27.0688 [°]
28.1440 [°]
28.5763 [°]
29.5035 [°]
30.3890 [°]
31.4548 [°]
42.4123 [°]
45.0433 [°]
10
20
30
40
50
60
70
Position [°2Theta] (COPPER(Cu))

Fig. 12

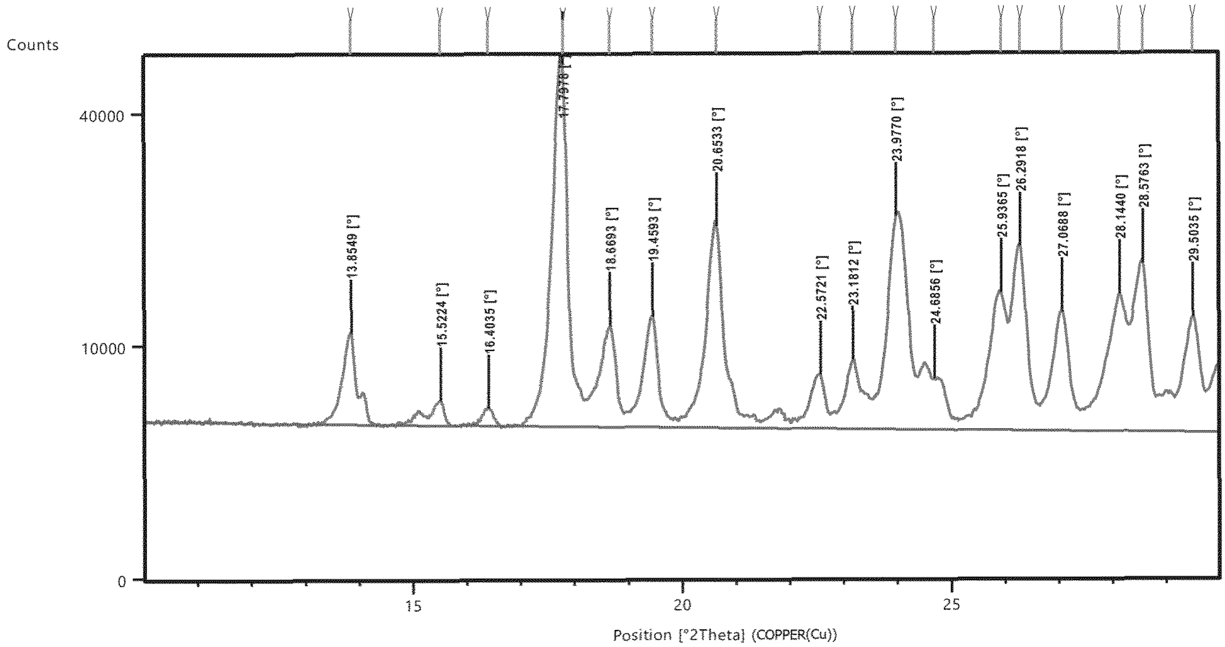
Counts
40000
10000
0
13.8549 [°]
15.5224 [°]
16.4035 [°]
17.7978 [°]
18.6693 [°]
19.4593 [°]
20.6533 [°]
22.5721 [°]
23.1812 [°]
23.9770 [°]
24.6856 [°]
25.9365 [°]
26.2918 [°]
27.0688 [°]
28.1440 [°]
28.5763 [°]
29.5035 [°]
15
20
25
Position [°2Theta] (COPPER(Cu))

# Fig. 13

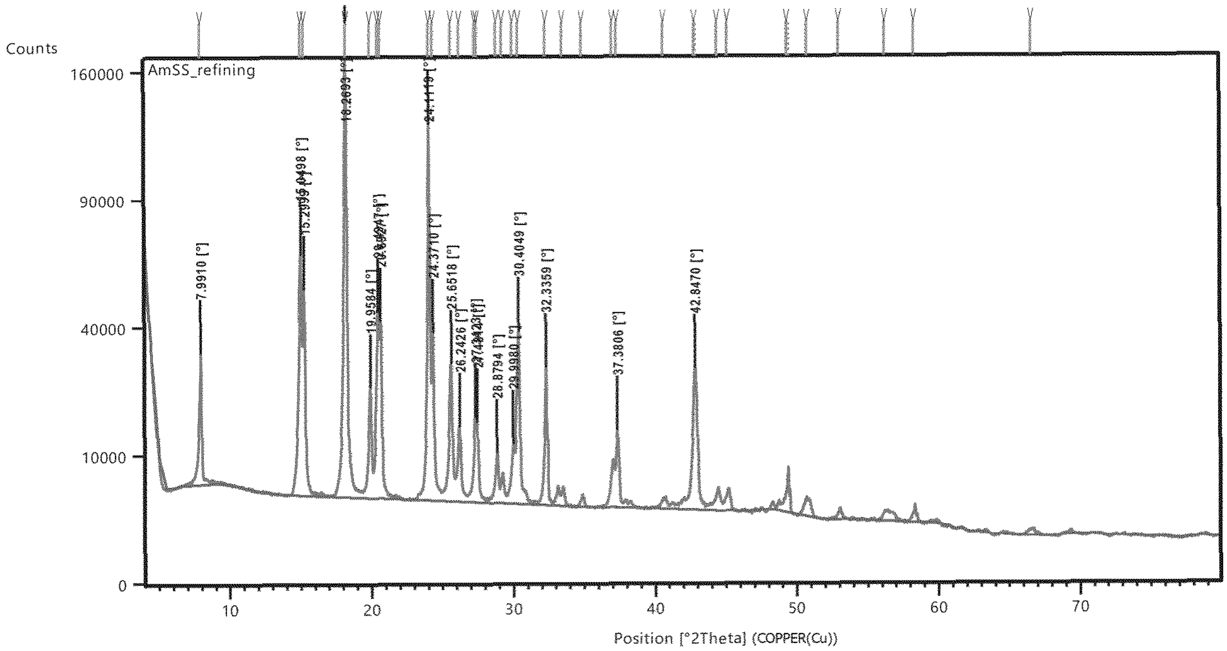
Counts
160000
90000
40000
10000
0
AmSS_refining
7.9910 [°]
18.2693 [°]
19.9584 [°]
24.1119 [°]
24.3710 [°]
25.6518 [°]
26.2426 [°]
28.8794 [°]
29.9980 [°]
30.4049 [°]
32.3359 [°]
37.3806 [°]
42.8470 [°]
10
20
30
40
50
60
70
Position [°2Theta] (COPPER(Cu))

# Fig. 14

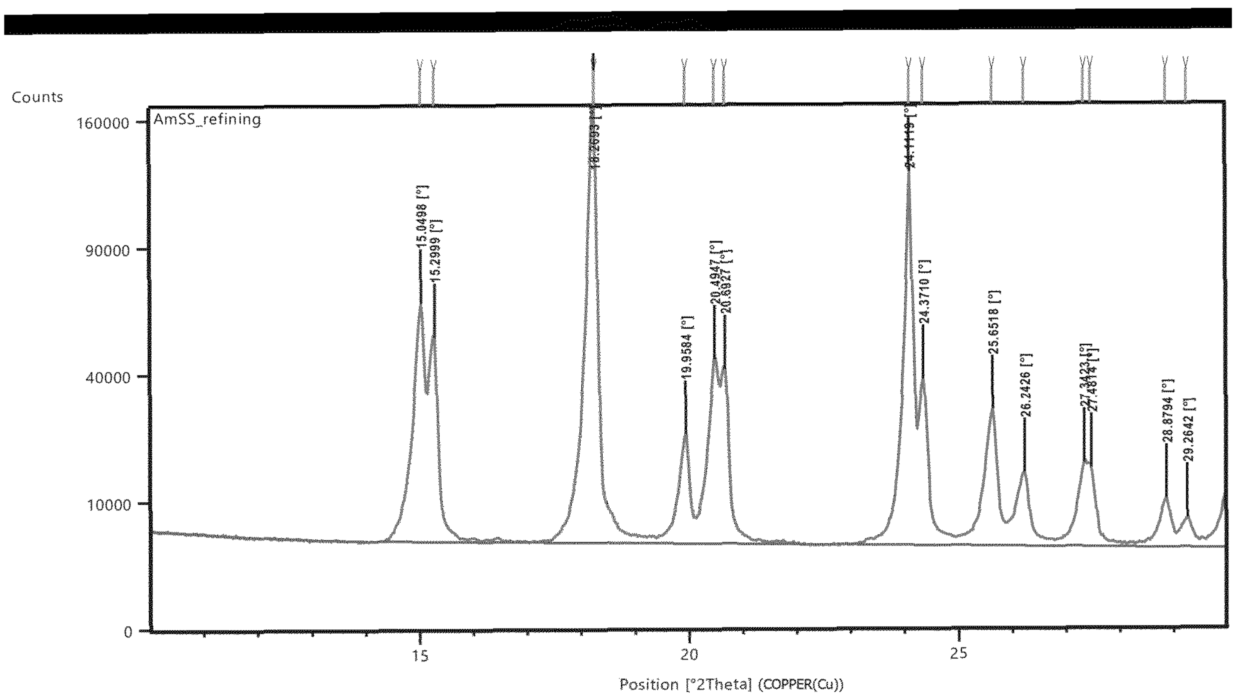
Counts
160000
90000
40000
10000
0
AmSS_refining
15.0498 [°]
15.2999 [°]
18.2693 [°]
19.9584 [°]
20.4947 [°]
20.6927 [°]
24.1119 [°]
24.3710 [°]
25.6518 [°]
26.2426 [°]
27.3423 [°]
27.4814 [°]
28.8794 [°]
29.2642 [°]
15
20
25
Position [°2Theta] (COPPER(Cu))

**INTERNATIONAL SEARCH REPORT**

International application No.
**PCT/JP2024/005404**

**A. CLASSIFICATION OF SUBJECT MATTER**

***C07C 309/29***(2006.01)i; ***C07C 303/42***(2006.01)i; ***C07C 309/30***(2006.01)i; ***C08F 12/30***(2006.01)i
FI: C07C309/29; C08F12/30; C07C309/30; C07C303/42

According to International Patent Classification (IPC) or to both national classification and IPC

**B. FIELDS SEARCHED**

Minimum documentation searched (classification system followed by classification symbols)

C07C309/29; C07C303/42; C07C309/30; C08F12/30

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | 服部達夫ほか. 臭素の有効利用研究(第2報) p-スチレンスルホン酸ナトリウム及びp-スチレンスルホン酸アンモニウムの製造と利用. 東洋曹達研究報告. vol. 24, no. 1, 1980, pp. 3-12, (HATTORI, Tatsuo et al. Studies on the Further New Uses of Bromine (Part II): Sodium and Ammonium p-Styrenesulfonates; Preparation and Uses. Scientific Report of Toyo Soda Manufacturing Company, Ltd.)<br>p. 5, left column, line 32 to p. 8, right column, line 5 from the bottom | 1-11 |
| A | | 12-15 |
| X | JP 2022-160712 A (TOSOH FINECHEM CORP.) 20 October 2022 (2022-10-20)<br>paragraphs [0031]-[0036] | 1, 4-8, 10-11 |
| A | | 2-3, 9, 12-15 |
| A | JP 50-149642 A (TOYO SODA MFG CO., LTD.) 29 November 1975 (1975-11-29)<br>claims, comparative examples | 1-15 |
| A | JP 56-102986 A (TOYO SODA MFG CO., LTD.) 17 August 1981 (1981-08-17)<br>claims, examples | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 April 2024** | **07 May 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/005404**

**C. DOCUMENTS CONSIDERED TO BE RELEVANT**

| Category* | Citation of document, with indication, where appropriate, of the relevant passages | Relevant to claim No. |
|---|---|---|
| A | JP 2015-209374 A (ASAHI KASEI CHEMICALS CORPORATION) 24 November 2015 (2015-11-24)<br>claims, paragraphs [0012]-[0016] | 1-15 |
| A | JP 2017-61422 A (TOSOH ORGANIC CHEMICAL CO.,LTD.) 30 March 2017 (2017-03-30)<br>claims, paragraphs [0044], [0045] | 1-15 |
| A | JP 2022-25184 A (TOSOH FINECHEM CORP.) 10 February 2022 (2022-02-10)<br>claims, paragraphs [0015], [0016] | 1-15 |
| P, X<br>P, A | JP 2023-155952 A (TOSOH FINECHEM CORP.) 24 October 2023 (2023-10-24)<br>claims, paragraph [0042] | 7-11<br>1-6, 12-15 |

Form PCT/ISA/210 (second sheet) (July 2022)

**INTERNATIONAL SEARCH REPORT**
**Information on patent family members**

International application No.
**PCT/JP2024/005404**

| Patent document cited in search report | Publication date (day/month/year) | Patent family member(s) | Publication date (day/month/year) |
|---|---|---|---|
| JP 2022-160712 A | 20 October 2022 | (Family: none) | |
| JP 50-149642 A | 29 November 1975 | US 4029640 A<br>claims, comparative examples | |
| JP 56-102986 A | 17 August 1981 | (Family: none) | |
| JP 2015-209374 A | 24 November 2015 | (Family: none) | |
| JP 2017-61422 A | 30 March 2017 | (Family: none) | |
| JP 2022-25184 A | 10 February 2022 | (Family: none) | |
| JP 2023-155952 A | 24 October 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3585588 B
- JP 3460246 B
- JP 5482194 B
- JP 6618355 B
- JP 2021044537 A
- WO 2020184306 A
- JP 50149642 A
- JP 51026842 A
- JP 10152465 A
- JP 2014080505 A
- US 6221248 B


### Non-patent literature cited in the description


- **JOSE M.ASUA**. *European Polymer Journal*, 2017, vol. 93, 480-494
- *Toyo Soda Research Report*, 1980, vol. 24 (1), 3-11
- **J.C.SALAMONE**. *Polymer Letters Edition*, 1977, vol. 15, 487-491
- **TERUO SUZUKI**. *Proceedings of the Institute of Electrostatics Japan*, 2001, vol. 1 (25), 37-44
- **KEISUKE TSUJI**. *Pulverization*, 2014, vol. 5, 84-88
- **KYOICHI SAITO**. Polymer Adsorbent by Graft Polymerization. Maruzen Publishing Co., 2014, 8-10